(19) Europäisches Patentamt
European Patent Office
Office européen des brevets

(11) Publication number : **0 126 546 B2**

(12) **NEW EUROPEAN PATENT SPECIFICATION**

(45) Date of publication of the new patent specification : **30.03.94 Bulletin 94/13**

(21) Application number : **84302584.2**

(22) Date of filing : **16.04.84**

(51) Int. Cl.$^5$ : **C12N 15/82,** C12N 1/20, A01H 1/00, // (C12N1/20, C12R1:01)

# (54) Plant structural gene expression.

(30) Priority : **15.04.83 US 485614**
**13.04.84 EP 84302535**

(43) Date of publication of application : **28.11.84 Bulletin 84/48**

(45) Publication of the grant of the patent : **13.12.89 Bulletin 89/50**

(45) Mention of the opposition decision : **30.03.94 Bulletin 94/13**

(84) Designated Contracting States : **AT BE CH DE FR GB IT LI LU NL SE**

(56) References cited :
**EP-A- 0 122 791**
**WO-A-84/02913**
**SCIENCE, vol. 222, November 4, 1983, pages 476-482; N.MURAI et al.:"Phaseolin Gene from Bean Is Expressed After Transfer to Sunflower Via Tumor-Inducing Plasmid Vectors".**
**CHEMICAL ABSTRACTS, vol. 101, no. 3, July 16, 1984, page 176, no. 18452n, Columbus, Ohio, US, J.D. KEMP et al.: "Transfer of a functional gene via the Tiplasmid"**
**MIAMI WINTER SYMPOSIA, vol. 19, "FROM GENE TO PROTEIN: TRANSLATION INTO BIOTECHNOLOGY", ed. by F.AHMAD et al., Academic Press 1982, NEW YORK, (US), page 514; P.V. CHOUDARY et al.: "Studies on expression of the phaseolin gene of french bean seeds in sunflower plant cells"**

(56) References cited :
**SCIENCE, vol.218, November 26, 1982, pages 854-859; L.W. REAM et al.: "Crown Gall Disease and Prospects for Genetic Manipulation of Plants"**
**Genetic Engineering: Applications to Agriculture; invited papers for symposium, 16-19 May 1982, Beltsville Agricultural Research Center; Proceedings published by Rowman & Allenhald, 1983, p. 215-228, J.D. Kemp et al.**
**Nature, 1981, 289: 37-41; Sun et al.**

(73) Proprietor : **LUBRIZOL GENETICS INC.**
**3375 Mitchell Lane**
**Boulder Colorado 80301-2244 (US)**

(72) Inventor : **Kemp, John D.**
**2514 NcKenna Boulevard**
**Madison Wisconsin 53711 (US)**
Inventor : **Hall, Timothy C.**
**210 North Whitney Way**
**Madison Wisconsin 53711 (US)**
Inventor : **Slightom, Jerry L.**
**5010 Retana Drive**
**Madison Wisconsin 53558 (US)**
Inventor : **Sutton, Dennis**
**5611 Alben Avenue**
**McFarland Wisconsin 53558 (US)**
Inventor : **Murai, Norimoto**
**138 West Gorham Street**
**Madison Wisconsin 53703 (US)**

(74) Representative : **Fisher, Adrian John et al**
**CARPMAELS & RANSFORD 43 Bloomsbury Square**
**London WC1A 2RA (GB)**

EP 0 126 546 B2

## Description

Shuttle vectors, developed by Ruvkun and Ausubel (1981) Nature *289*:85-88, provide a way to insert foreign genetic materials into positions of choice in a large plasmid, virus, or genome. There are two main problems encountered when dealing with large plasmids or genomes. Firstly, the large plasmids may have many sites for each restriction enzyme. Unique, site-specific cleavage reactions are not reproducible and multi-site cleavage reactions followed by ligation lead to great difficulties due to the scrambling of the many fragments whose order and orientation one does not want changed. Secondly, the transformation efficiency with large DNA plasmids is very low. Shuttle vectors allow one to overcome these difficulties by facilitating the insertion, often *in vitro,* of the foreign genetic material into a smaller plasmid, then transferring, usually by *in vivo* techniques, to the larger plasmid.

A shuttle vector consists of a DNA molecule, usually a plasmid, capable of being introduced into the ultimate recipient bacteria. It also includes a copy of the fragment of the recipient genome into which the foreign genetic material is to be inserted and a DNA segment coding for a selectable trait, which is also inserted into the recipient genome fragment. The selectable trait ("marker") is conveniently inserted by transposon mutagenesis or by restriction enzymes and ligases.

The shuttle vector can be introduced into the ultimate recipient cell typically a bacterium of the genus *Agrobacterium* by a tri-parental mating (Ruvkun and Ausubel, *supra*.), direct transfer of a self-mobilizable vector in a bi-parental mating, direct uptake of exogenous DNA by *Agrobacterium* cells ("transformation", using the conditions of M. Holsters *et al.* (1978) Molec. Gen. Genet. 163:181-187), by spheroplast fusion of *Agrobacterium* with another bacterial cell, by uptake of liposome-encapsulated DNA, or infection with a shuttle vector that is based on a virus that is capable of being packaged *in vitro.* A tri-parental mating involves the mating of a strain containing a mobilizable plasmid, which carries genes for plasmid mobilization and conjugative transfer, with the strain containing the shuttle vector. If the shuttle vector is capable of being mobilized by the plasmid genes, the shuttle vector is transferred to the recipient cell containing the large genome, e.g. the Ti or Ri plasmids of *Agrobacterium* strains.

After the shuttle vector is introduced into the recipient cell, possible events include a double cross over with one recombinational event on either side of the marker. This event will result in transfer of a DNA segment containing the marker to the recipient genome replacing a homologous segment lacking the insert. To select for cells that have lost the original shuttle vector, the shuttle vector must be incapable of replicating in the ultimate host cell or be incompatible with an independently selectable plasmid preexisting in the recipient cell. One common means of arranging this is to provide in the third parent another plasmid which is incompatible with the shuttle vector and which carries a different drug resistance marker. Therefore, when one selects for resistance to both drugs, the only surviving cells are those in which the marker on the shuttle vector has recombined with the recipient genome. If the shuttle vector carries an extra marker, one can then screen for and discard cells that are the result of a single cross-over between the shuttle vector and the recipient plasmid resulting in cointegrates in which the entire shuttle vector is integrated with the recipient plasmid. If the foreign genetic material is inserted into or adjacent to the marker that is selected for, it will also be integrated into the recipient plasmid as a result of the same double recombination. It might also be carried along when inserted into the homologous fragment at a spot not within or adjacent to the marker, but the greater the distance separating the foreign genetic material from the marker, the more likely will be a recombinational event occurring between the foreign genetic material and marker, preventing transfer of the foreign genetic material.

Shuttle vectors have proved useful in manipulation of *Agrobacterium* plasmids: see D. J. Garfinkel *et al.* (1981) Cell *27*:143-153, A. J. M. Matzke and M. D. Chilton (1981) J. Molec. Appl. Genet. *1*:39-49, and J. Leemans *et al.* (1981) J. Molec. Appl. Genet. *1*:149-164, who referred to shuttle vectors by the term "intermediate vectors".

### *Agrobacterium*-Overview

Included within the gram-negative bacterial family Rhizobiaceae in the genus *Agrobacterium* are the species *A. tumefaciens* and *A. rhizogenes.* These species are respectively the causal agents of crown gall disease and hairy root disease of plants. Crown gall is characterized by the growth of a gall of dedifferentiated tissue. Hairy root is a teratoma characterized by inappropriate induction of roots in infected tissue. In both diseases, the inappropriately growing plant tissue usually produces one or more amino acid derivatives, known as opines, not normally produced by the plant which are catabolized by the infecting bacteria. Known opines have been classified into three families whose type members are octopine, nopaline, and agropine. The cells of inappropriately growing tissues can be grown in culture, and, under appropriate conditions, be regenerated into whole plants that retain certain transformed phenotypes.

Virulent strains of *Agrobacterium* harbor large plasmids known as Ti (tumor-inducing) plasmids in *A. tumefaciens* and Ri (root-inducing) plasmids in *A. rhizogenes.* Curing a strain of these plasmids results in a loss of pathogenicity. The Ti plasmid contains a region, referred to as T-DNA (transferred-DNA), which in tumors is found to be integrated into the genome of the host plant. The T-DNA encodes several transcripts. Mutational studies have shown that some of these are involved in induction of tumorous growth. Mutants in the genes for *tml, tmr,* and *tms,* respectively, result in large tumors (in tobacco), a propensity to generate roots, and a tendency for shoot induction. The T-DNA also encodes the gene for at least one opine synthetase, and the Ti plasmids are often classified by the opine which they caused to be synthesized. Each of the T-DNA genes is under control of a T-DNA promoter. The T-DNA promoters resemble eukaryotic promoters in structure, and they appear to function only in the transformed plant cell. The Ti plasmid also carries genes outside the T-DNA region. These genes are involved in functions which include opine catabolism, oncogenicity, agrocin sensitivity, replication, and autotransfer to bacterial cells. The Ri plasmid is organized in a fashion analogous to the Ti plasmid. The set of genes and DNA sequences responsible for transforming the plant cell are hereinafter collectively referred to as the transformation-inducing principle (TIP). The designation TIP therefore includes both Ti and Ri plasmids. The integrated segment of a TIP is termed herein "T-DNA", whether derived from a Ti plasmid or an Ri plasmid. Recent general reviews of *Agrobacterium*-caused disease include those by D. J. Merlo (1982), Adv. Plant Pathol. *1*:139-178 L. W. Ream and M. P. Gordon (1982), Science *218*:854-859, and M. W. Bevan and M. D. Chilton (1982), Ann. Rev. Genet. *16*:357-384; G. Kahl and J. Schell (1982) *Molecular Biology of Plant Tumors.*

*Agrobacterium*-Infection of plant tissues

Plant cells can be transformed by *Agrobacterium* in a number of methods known in the art which include but are not limited to co-cultivation of plant cells in culture with *Agrobacterium,* direct infection of a plant, fusion of plant protoplasts with *Agrobacterium* spheroplasts, direct transformation by uptake of free DNA by plant cell protoplasts, transformation of protoplasts having partly regenerated cell walls with intact bacteria, transformation of protoplasts by liposomes containing T-DNA, use of a virus to carry in the T-DNA, microinjection, and the like. Any method will suffice as long as the gene is reliably expressed, and is stably transmitted through mitosis and meiosis.

The infection of plant tissue by *Agrobacterium* is a simple technique well known to those skilled in the art (for an example, see D. N. Butcher *et al.* (1980) in *Tissue Culture Methods for Plant Pathologists,* eds.: D. S. Ingrams and J. P. Helgeson, pp. 203-208). Typically a plant is wounded by any of a number of ways, which include cutting with a razor, puncturing with a needle, or rubbing with abrasive. The wound is then inoculated with a solution containing tumor-inducing bacteria. An alternative to the infection of intact plants is the inoculation of pieces of tissues such as potato tuber disks (D. K. Anand and G. T. Heberlein (1977) Amer. J. Bot. *64*:153-158) or segments of tobacco stems (K. A. Barton, *et al.* (1983) Cell *32*:1033-1043). After induction, the tumors can be placed in tissue culture on media lacking phytohormones. Hormone independent growth is typical of transformed plant tissue and is in great contrast to the usual conditions of growth of such tissue in culture (A. C. Braun (1956) Cancer Res. *16*:53-56).

*Agrobacterium* is also capable of infecting isolated cells and cells grown in culture, Marton *et al.* (1979) Nature *277*:129-131, and isolated tobacco mesophyll protoplasts. In the latter technique, after allowing time for partial regeneration of new cell walls, *Agrobacterium* cells were added to the culture for a time and then killed by the addition of antibiotics. Only those cells exposed to *A. tumefaciens* cells harboring the Ti plasmid were capable of forming calli when plated on media lacking hormone. Most calli were found to contain an enzymatic activity involved in opine anabolism. Other workers (R. B. Horsch and R. T. Fraley (18 January 1983) 15th Miami Winter Symposium) have reported transformations by co-cultivation, leading to a high rate (greater than 10%) of calli displaying hormone-independent growth, with 95% of those calli making opines. M. R. Davey *et al.* (1980) in Ingram and Helgeson, *supra,* pp. 209-219, describe the infection of older cells that had been regenerated from protoplasts.

Plant protoplasts can be transformed by the direct uptake of TIP plasmids. M. R. Davey *et al.* (1980) Plant Sci. Lett. *18*:307-313, and M. R. Davey *et al.* (1980) in Ingram and Helgeson, *supra,* were able to transform *Petunia* protoplasts with the Ti plasmid in the presence of poly-L-*alpha*-ornithine to a phenotype of opine synthesis and hormone-independent growth in culture. It was later shown (J. Draper *et al.* (1982) Plant and Cell Physiol. *23*:451-458, M. R. Davey *et al.* (1982) in *Plant Tissue Culture 1982*, ed: A. Fujiwara, pp. 515-516) that polyethylene glycol stimulated Ti uptake and that some T-DNA sequences were integrated into the genome. F. A. Krens *et al.* (1982) Nature *296*:72-74, reported similar results using polyethylene glycol following by a calcium shock, though their data suggests that the integrated T-DNA included flanking Ti plasmid sequences.

An alternative method to obtain DNA uptake involves the use of liposomes. The preparation of DNA containing liposomes is taught by Papahadjopoulos in US Patents 4,078,052 and 4,235,871. Preparations for the introduction of Ti-DNA *via* liposomes have been reported (T. Nagata *et al.* (1982) in Fujiwara, *supra,* pp. 509-510, and T. Nagata (1981) Mol. Gen. Genet. *184*:161-165). An analogous system involves the fusion of plant and bacterial cells after removal of their cell walls. An example of this technique is the transformation of *Vinca* protoplast by *Agrobacterium* spheroplasts reported by S. Hasezawa *et al.* (1981) Mol. Gen. Genet. *182*:206-210. Plant protoplasts can take up cell wall delimited *Agrobacterium* cells (S. Hasezawa *et al.* (1982) in Fujiwara, *supra* pp. 517-518).

T-DNA can be transmitted to tissue regenerated from a fusion of two protoplasts, only one of which had been transformed (G. J. Wullems *et al.* (1980) Theor. Appl. Genet. *56*:203-208). As detailed in the section on Regeneration of Plants, T-DNA can pass through meiosis and be transmitted to progeny as a simple Mendelian trait.

*Agrobacterium*-Regeneration of plants

Differentiated plant tissues with normal morphology have been obtained from crown gall tumors. A. C. Braun and H. N. Wood (1976) Proc. Natl. Acad. Sci. USA *73*:496-500, grafted tobacco teratomas onto normal plants and were able to obtain normally appearing shoots which could flower. The shoots retained the ability to make opines and to grow independently of phytohormones when placed in culture. In the plants screened, these tumor phenotypes were not observed to be transmitted to progeny, apparently being lost during meiosis (R. Turgeon *et al.* (1976) Proc. Nat. Acad. Sci. USA *73*:3562-3564). Plants which had spontaneously lost tumorous properties, or which were derived from teratoma seed, were initially shown to have lost all their T-DNA (F.-M. Yang *et al.* (1980) In Vitro *16*:87-92, F. Yang *et al.* (1980) Molec. Gen. Genet. *177*:707-714, M. Lemmers *et al.* (1980) J. Mol. Biol. *144*:353-376). However, later work with plants that had become revertants after hormone treatment (1 mg/l kinetin) showed that plants which had gone through meiosis, though losing T-DNA genes responsible for the transformed phenotype, could retain sequences homologous to both ends of T-DNA (F. Yang and R. B. Simpson (1981) Proc. Nat. Acad. Sci. USA *78*:4151-4155). G. J. Wullems *et al.* (1981) Cell *24*:719-724, further demonstrated that genes involved in opine anabolism were capable of passing through meiosis though the plants were male sterile and that seemingly unaltered T-DNA could be inherited in a Mendelian fashion (G. Wullems *et al.* (1982) in A. Fujiwara, *supra*). L. Otten *et al.* (1981) Molec. Gen. Genet. *183*:209-213, used Tn7 transposon-generated Ti plasmid mutants in the *tms* (shoot-inducing) locus to create tumors which proliferated shoots. When these shoots were regenerated into plants, they were found to form self-fertile flowers. The resultant seeds germinated into plants which contained T-DNA and made opines. Similar experiments with a *tmr* (root-inducing) mutant showed that full-length T-DNA could be transmitted through meiosis to progeny, that in those progeny nopaline genes could be expressed, though at variable levels, and that the cotransformed yeast alcohol dehydrogenase I gene was not expressed (K. A. Barton *et al.* (1983) (Cell *32*:1033-1043). It now appears that regenerated tissues which lack T-DNA sequences are probably descended from untransformed cells which "contaminate" the tumor (G. Ooms *et al.* (1982) Cell *30*:589-597).

Roots resulting from transformation from *A. rhizogenes* have proven relatively easy to regenerate into plantlets (M.-D. Chilton *et al.* (1982) Nature 295:432-434.

*Agrobacterium*-Genes on the TIP plasmids:

A number of genes have been identified within the T-DNA of the TIP plasmids. About half a dozen octopine plasmid T-DNA transcripts have been mapped (S. B. Gelvin *et al.* (1982) Proc. Natl. Acad. Sci. USA *79*:76-80, L. Willmitzer *et al.* (1982) EMBO J. *1*:139-146) and some functions have been assigned (J. Leemans *et al.* (1982) EMBO J. *1*:147-152). The four genes of an octopine type plasmid that have been well defined by transposon mutagenesis include *tms, tmr,* and *tml* (D. J. Garfinkel *et al.* (1981) Cell *27*:143-153). Ti plasmids which carry mutations in these genes respectively incite tumorous calli of *Nicotiana tabacum* which generate shoots, proliferate roots, and are larger than normal. In other hosts, mutants of these genes can induce different phenotypes (see Bevan and Chilton, *supra.*). The phenotypes of *tms* and *tmr* are correlated with differences in the phytohormone levels present in the tumor. The differences in cytokinin:auxin ratios are similar to those which in culture induce shoot or root formation in untransformed callus tissue (D. E. Akiyoshi *et al.* (1983) Proc. Natl. Acad. Sci. USA *80*:407-411). T-DNA containing a functional gene for either *tms* or *tmr* alone, but not functional *tml* alone, can promote significant tumor growth. Promotion of shoots and roots is respectively stimulated and inhibited by functional *tml* (L. W. Ream *et al.* (1983) Proc. Natl. Acad. Sci. USA *80*:1660-1664). Mutations in T-DNA genes do not seem to affect the insertion of T-DNA into the plant genome (J. Leemans *et al.* (1982) *supra*, L. W. Ream *et al.* (1983) *supra).* The *ocs* gene encodes octopine synthetase, which has been sequenced by H. De Greve *et al.* (1982) J. Mol. Appl. Genet. *1*:499-511. It does not contain introns (invervening sequences commonly found in eukaryotic genes which are posttranscriptionally spliced out of the messenger precursor during maturation of the mRNA). It does have sequences that resemble a eukaryotic transcriptional signal ("TATA box") and a polyadenylation site. As plant cells containing the enzyme octopine synthetase detoxify homo-arginine, the *ocs* gene may prove to be a useful selectable marker for plant cells that have been transformed by foreign DNA (G. M. S. Van Slogteren *et al* (1982) Plant Mol. Biol. *1*:133-142).

Nopaline Ti plasmids encode the nopaline synthetase gene *(nos),* which has been sequenced by A. Depicker *et al.* (1982) J. Mol. Appl. Genet. *1*:561-573. As was found with the *ocs* gene, *nos* is not interrupted by introns. It has two putative polyadenylation sites and a potential "TATA box". In contrast to *ocs, nos* is preceded by a sequence which may be a transcriptional signal known as a "CAT box". J. C. McPhersson *et al.* (1980) Proc. Natl. Acad. Sci. USA 77:2666-2670, reported the *in vitro* translation of T-DNA encoded mRNAs from crown gall tissues.

Transcription from hairy root T-DNA has also been detected (L. Willmitzer *et al.* (1982) Mol. Gen. Genet. *186*:16-22). Functionally, the hairy root syndrome appears to be equivalent of a crown gall tumor incited by a Ti plasmid mutated in *tmr* (F. F. White and E. W. Nester (1980) J. Bacteriol. *144*:710-720.

In eukaryotes, methylation (especially of cytosine residues) of DNA is correlated with transcriptional inactivation; genes that are relatively undermethylated are transcribed into mRNA. Gelvin *et al.* (1983) Nucleic Acids Res. *1*:159-174 have found that the T-DNA in crown gall tumors is always present in at least one unmethylated copy. That the same genome may contain numerous other copies of T-DNA which are methylated suggests that the copies of T-DNA in excess of one may be biologically inert. (See also G. Ooms *et al.* (1982) Cell *30*:589-597).

The Ti plasmid encodes other genes which are outside of the T-DNA region and are necessary for the infection process. (See M. Holsters *et al.* (1980) Plasmid *3*:212-230 for nopaline plasmids, and H. De Greve *et al.* (1981) Plasmid *6*:235-248, D. J. Garfinkel and E. W. Nester (1980) J. Bacteriol *144*:732-743, and G. Oorns (1980) J. Bacteriol *144*:82-91 for octopine plasmids). Most important are the *onc* genes, which when mutated result in Ti plasmids incapable of oncogenicity. (These loci are also known as *vir,* for virulence). The *onc* genes function in *trans,* being capable of causing the transformation of plant cells with T-DNA of a different plasmid type and physically located on another plasmid (J. Hille *et al.* (1982) Plasmid *7*:107-118, H. J. Klee *et al.* (1982) J. Bacteriol. *150*:327-331, M.-D. Chilton (18 January 1983) 15th Miami Winter Symp. Nopaline Ti DNA has direct repeats of about 25 base pairs immediately adjacent to the left and right borders of the T-DNA which might be involved in either excision from the Ti plasmid or integration into the host genome (N. S. Yadav *et al.* (1982) Proc. Natl. Acad. Sci. USA *79*:6322-6326), and a homologous sequence has been observed adjacent to an octopine T-DNA border (R. B. Simpson *et al.* (1982) Cell *29*:1005-1014). Opine catabolism is specified by the *ocs* and *nos* genes, respectively of octopine- and nopaline-type plasmids. The Ti plasmid also encodes functions necessary for its own reproduction including an origin of replication. Ti plasmid transcripts have been detected in *A. tumefaciens* cells by S. B. Gelvin *et al.* (1981) Plasmid *6*:17-29, who found that T-DNA regions were weakly transcribed along with non-T-DNA sequences. Ti plasmid-determined characteristics have been reviewed by Merlo, *supra* (see especially Table II), and Ream and Gordon *supra.*

*Agrobacterium*-TIP Plasmid DNA

Different octopine-type Ti plasmids are nearly 100% homologous to each other when examined by DNA hybridization (T. C. Currier and E. W. Nester (1976) J. Bacteriol. *126*:157-165) or restriction enzyme analysis (D. Sciaky *et al.* (1978) Plasmid *1*:238-253). Nopaline-type Ti plasmids have as little as 67% homology to each other (Currier and Nester, *supra).* A survey revealed that different Ri plasmids are very homologous to each other (P. Costantino *et al.* (1981) Plasmid *5*:170-182). N. H. Drummond and M.-D. Chilton (1978) J. Bacteriol. *136*:1178-1183, showed that proportionally small sections of octopine and nopaline type Ti plasmids were homologous to each other. These homologies were mapped in detail by G. Engler *et al.* (1981) J. Mol. Biol. *152*:183-208. They found that three of the four homologous regions were subdivided into three (overlapping the T-DNA), four (containing some *onc* genes), and nine (having *onc* genes) homologous sequences. The uninterrupted homology contains at least one *tra* gene (for conjugal transfer of the Ti plasmid to other bacterial cells), and genes involved in replication and incompatibility. This uninterrupted region has homology with a *Sym* plasmid (involved in symbiotic nitrogen fixation) from a species of *Rhizobium,* a different genus in the family Rhizobiaceae (R. K. Prakash *et al.* (1982) Plasmid *7*:271-280). The order of the four regions is not conserved, though they are all oriented in the same direction. Part of the T-DNA sequence is very highly conserved between nopaline and octopine plasmids (M.-D. Chilton *et al.* (1978) Nature *275*:147-149, A. Depicker *et al.* (1978) Nature *275*:150-153). Ri plasmids have been shown to have extensive homology among themselves, and to both octopine (F. F. White and E. W. Nester (1980) J. Bacteriol. *144*:710-720) and nopaline (G. Risuleo *et al* (1982) Plasmid *7*:45-51) Ti plasmids, primarily in regions encoding *onc* genes. Ri T-DNA contains extensive though weak homologies to T-DNA from both types of Ti plasmid (L Willmitzer *et al.* (1982) Mol. Gen. Genet. *186*:3193-3197). Plant DNA from uninfected *Nicotiana glauca* contains sequences, referred to as cT-DNA (cellular T-DNA), that show homology to a portion of the Ri T-DNA (F. F. White *et al.* (1983) Nature *301*:348-350).

It has been shown that a portion of the Ti (M.-D. Chilton *et al.* (1977) Cell *11*:263-271) or Ri (M.-D. Chilton (1982) Nature *295*:432-434, F. F. White *et al.* (1982) Proc. Natl. Acad. Sci. USA *79*:3193-3197, L. Willmitzer (1982) Mol. Gen. Genet. *186*:16-22) plasmid is found in the DNA of tumorous plant cells. The transferred DNA is known as T-DNA. T-DNA is integrated into the host DNA (M. F. Thomashow *et al. (1980)* Proc. Natl. Acad. Sci. USA *77*:6448-6452, N. S. Yadav *et al.* (1980) Nature *287*:456-461) in the nucleus (M. P. Nuti *et al.* (1980) Plant Sci. Lett. *18*:1-6, L. Willmitzer *et al.* (1980) Nature *287*:359-361, M.-D. Chilton *et al.* (1980) Proc. Natl. Acad. Sci. USA *77*:4060-4064).

M. F. Thomashow *et al.* (1980) Proc. Natl. Acad. Sci. USA *77*:6448-6452, and M. F. Thomashow *et al.* (1980) Cell *19*:729-739, found the T-DNA from octopine-type Ti plasmids to have been integrated in two separate sections, TL-DNA and TR-DNA, left and right T-DNAs respectively. The copy numbers of TR and TL can vary (D. J. Merlo *et al.* (1980) *M*olec. Gen. Genet. *177*:637-643). A core of T-DNA is highly homologous to nopaline T-DNA (Chilton *et al.* (1978) *supra* and Depicker *et al.* (1978) *supra),* is required for tumor maintenance, is found in TL, is generally present in one copy per cell, and codes for the genes *tms, tmr* and *tml.* On the other hand TR can be totally dispensed with (M. De Beuckeleer *et al.* (1981) Molec. Gen. Genet. *183*:283-288, G. Ooms *et al.* (1982) Cell *30*:589-597), though found in a high copy number (D. J. Merlo et *al.* (1980) *supra).* G. Ooms et *al.* (1982) Plasmid *7*:15-29, hypothesized that TR is involved in T-DNA integration, though they find that when TR is deleted from the Ti plasmid, *A. tumefaciens* does retain some virulence. G. Ooms *et al.* (1982) Cell *30*:589-597, showed that though T-DNA is occasionally deleted after integration in the plant genome, it is generally stable and that tumors containing a mixture of cells that differ in T-DNA organization are the result of multiple transformation events. The *ocs* is found in TL but can be deleted from the plant genome without loss of phenotypes related to tumorous growth. The left border of integrated TL has been observed to be composed of repeats of T-DNA sequences which are in either direct or inverted orientations (R. B. Simpson *et al.* (1982) Cell *29*:1005-1014).

In contrast to the situation in octopine-type tumors, nopaline T-DNA is integrated into the host genome in one continuous fragment (M. Lemmers *et al.* (1980) J. Mol. Biol. *144*:353-376, P. Zambryski *et al.* (1980) Science *209*:1385-1391). Direct tandem repeats were observed. T-DNA of plants regenerated from teratomas had minor modifications in the border fragments of the inserted DNA (Lemmers *et al. supra).* Sequence analysis of the junction between the right and left borders revealed a number of direct repeats and one inverted repeat. The latter spanned the junction (Zambryski *et al.* (1980) *supra).* The left junction has been shown to vary by at least 70 base pairs while the right junction varies no more than a single nucleotide (P. Zambryski *et al.* (1982) J. Molec. Appl. Genet. *1*:361-370). Left and right borders in junctions of tandem arrays were separated by spacers which could be over 130 bp. The spacers were of unknown origin and contained some T-DNA sequences. T-DNA was found to be integrated into both repeated and $l_{ow}$ copy number host sequences.

N. S. Yadav *et al.* (1982) Proc. Natl. Acad. Sci. USA *79*:6322-6326, have found a *chi* site, which in the bacteriophage *lambda* augments general recombination in the surrounding DNA as far as 10 kilobases away, in a nopaline Ti plasmid just outside the left end of the T-DNA. R. B. Simpson *et al.* (1982) Cell *29*:1005-1014, have not observed a *chi* sequence in an octopine Ti plasmid, though the possible range of action does not eliminate the possibility of one being necessary and present but outside of the region sequenced. The significance of the *chi* in the Ti plasmid is not known. If the *chi* has a function, it is probably used in *Agrobacterium* cells and not in the plants, as *chi* is not found within the T-DNA.

*Agrobacterium*-Manipulations of the TIP plasmids

As detailed in the section on Shuttle Vectors, technology has been developed for the introduction of altered DNA sequences into desired locations on a TIP plasmid. Transposons can be easily inserted using this technology (D. J. Garfinkel *et al.* (1981) Cell *27*:143-153). J.-P. Hernalsteen *et al.* (1980) Nature *287*:654-656, have shown that a DNA sequence (here a bacterial transposon) inserted into T-DNA in the Ti plasmid is transferred and integrated into the recipient plant's genome. Though insertion of foreign DNA has been done with a number of genes from different sources, to date the genes have not been expressed under control of their own promoters. Sources of these genes include alcohol dehydrogenase (Adh) from yeast (K. A. Barton *et al.* (1983) Cell, *32*:1033-1043), Adhl (J. Bennetzen, unpublished) and zein from corn, interferon and globin from mammals, and the mammalian virus SV40 (J. Schell, unpublished). M. Holsters *et al.* (1982) Mol. Gen. Genet. *185*:283-289, have shown that a bacterial transposon (Tn7) inserted into T-DNA could be recovered in a fully functional and seemingly unchanged form after integration into a plant genome.

Deletions can be generated in a TIP plasmid by several methods. Shuttle vectors can be used to introduce deletions constructed by standard recombinant DNA techniques (Cohen and Boyer US Pat. 4,237,224). Deletions with one predetermined end can be created by the improper excision of transposons (B. P. Koekman *et al.* (1979) Plasmid *2*:347-357, G. Ooms *et al.* (1982) Plasmid *7*:15-29). J. Hille and R. Schilperoot (1981) Plasmid *6*:151-154, have demonstrated that deletions having both ends at predetermined positions can be generated by use of two transposons. The technique can also be used to construct "recombinant DNA" molecules *in vivo.*

The nopaline synthetase gene has been used for insertion of DNA segments coding for drug resistance that can be used to select for transformed plant cells. M. Bevan (reported by M.-D. Chilton *et al.* (18 January 1983) 15th Miami Winter Symp., see also J. L. Marx (1983) Science *219*:830) and R. Horsch *et al.* (18 January 1983) 15th Miami Winter Symp., see Marx, *supra,* have inserted the kanamycin resistance gene (neomycin phosphotransferase) from Tn5 behind (under control of) the nopaline promoter. The construction was used to transform plant cells which in culture displayed resistance to kanamycin and its analogs such as G418. J. Schell *et al.* (18 January 1983) 15th Miami Winter Symp. (see also Marx, *supra*), reported a similar construction, in which the methotrexate resistance gene (dihydrofolate reductase) from Tn7 was placed behind the nopaline synthetase promoter. Transformed cells were resistant to methotrexate. As plant cells containing octopine synthetase are resistant to the toxic chemical homo-arginine, G. M. S. Van Slogteren *et al.* (1982) Plant Mol. Biol. *1*:133-142, have proposed using that enzyme as a selectable marker.

M.-D. Chilton *et al.* (1983) *supra,* reported that A. de Framond has constructed a "mini-Ti plasmid". In the nopaline T-DNA there is normally only one site cut by the restriction enzyme *Kpn*I. A mutant lacking the site was constructed and a KpnI fragment, containing the entire nopaline T-DNA, was isolated. This fragment together with a kanamycin resistance gene was inserted into pRK290, thereby resulting in a plasmid which could be maintained in *A. tumefaciens* and lacked almost all non-T-DNA Ti sequences. By itself, this plasmid was not able to transform plant cells. However when placed in an *A. tumefaciens* strain containing an octopine Ti plasmid, tumors were induced which synthesized both octopine and nopaline. This indicated that the missing nopaline Ti plasmid functions were complemented by the octopine Ti plasmid, and that the nopaline "mini-Ti" was functional in the transformation of plant cells. Chilton *et al.* (1983) *supra* also reported on the construction of a "micro-Ti" plasmid made by resectioning the mini-Ti with *Sma*I to delete essentially all of T-DNA but the nopaline synthetase gene and the left and right borders. The micro-Ti was inserted into a modified pRK290 plasmid that was missing its *Sma*I site, and employed in a manner similar to mini-Ti, with comparable results.

H. Lorz *et al* (1982) in *Plant Tissue Culture 1982,* ed: A. Fujiwara, pp. 511-512, reported the construction of a plasmid vector, apparently independent of the TIP system for DNA uptake and maintenance, that used the nopaline synthetase gene as a marker.

Phaseolin and gene regulation

In general the genes of higher eukaryotes are highly regulated. A multicellular organism, such as a plant, has a number of differentiated tissues, each with its own specialized functions, each of which requires specialized gene products. One such tissue is the cotyledon. In legumes, the cotyledons usually serve as the storage tissue for the seed, holding reserves of lipid, carbohydrate. minerals, and protein until the seed needs them during germination. In *Phaseolus vulgaris* L. (also known as the French bean, kidney bean, navy bean, green bean and other names), the major storage protein is known as phaseolin. This protein comprises a small number of molecular species that are extremely homologous and equivalent to one another. Phaseolin contributes most of the nutrition value of dried beans, often comprising more than 10% of the weight of a dried bean.

Phaseolin is highly regulated during the life cycle of *P. vulgaris.* The protein is made essentially only while seed is developing within the pod. Levels rise from the limit of detection to as much as half the seed's protein content, following genetically determined schedules for synthesis. At its peak, phaseolin synthesis can account for over 80% of a cotyledon cell's protein synthesis. At other times and in other tissues, phaseolin synthesis is undetectable. The extreme nature of phaseolin's regulation, coupled with its worldwide nutritional importance, has led to much interest in the study of phaseolin, its properties, and its regulation.

Summary of the invention

The invention disclosed herein provides a plant comprising a genetically modified plant cell having a plant structural gene introduced and expressed therein under control of the promoter of the "1.6" transcript of T-DNA, the promoter and the plant structural gene being in such a position and orientation with respect to each other that the plant structural gene is expressible in the plant cell under control of the T-DNA promoter, and the plant structural gene comprising an intron. Also provided are novel strains of bacteria containing and replicating T-DNA, as defined herein, the T-DNA being modified to contain an inserted plant structural gene in such orientation and spacing with respect to the promoter of the "1.6" transcript of T-DNA as to be expressible in the plant cell under control of the T-DNA promoter. Further, the invention provides novel plasmids having the ability to replicate in *E. coli* and comprising T-DNA, and further comprising a plant structural gene inserted within T-DNA contained within the plasmid, in such a manner as to be expressible in a plant cell under control of the promoter of the "1.6" transcript of T-DNA, and the plant structural gene comprising an intron.

The experimental work disclosed herein is believed to be the first demonstration that plant structural genes are expressible in plant cells under control of a T-DNA promoter, after introduction via T-DNA, that is to say, by inserting the plant structural genes into T-DNA under control of a T-DNA promoter and introducing the T-DNA containing the insert into a plant cell using known means. The disclosed experiments are also believed to provide the first demonstration that plant structural genes containing introns are expressed in plant cells under control of a T-DNA promoter after introduction via T-DNA. These results are surprising in view of the fact that the genes previously reported to be expressible in T-DNA under control of a T-DNA promoter, either endogenous T-DNA genes or inserted foreign genes. lacked introns. The results are unexpected also in view of the prior art failure to demonstrate that a T-DNA promoter could function to control expression of a plant structural gene when the latter is introduced into T-DNA under the proper conditions. The invention is useful for genetically modifying plant tissues and whole plants by inserting useful plant structural genes from other plant species or strains. Such useful plant structural genes include, but are not limited to, genes coding for storage proteins, lectins, disease resistance factors, herbicide resistance factors, insect resistance factors, environmental stress tolerance factors, specific flavor elements, and the like. The invention is exemplified by introduction and expression of a structural gene for phaseolin, the major seed storage protein of the bean *Phaseolus vulgaris* L., into sunflower and tobacco plant cells. Once plant cells expressing a plant structural gene under control of a T-DNA promoter are obtained, plant tissues and whole plants can be regenerated therefrom using methods and techniques well known in the art. The regenerated plants are then reproduced by conventional means and the introduced genes can be transferred to other strains and cultivars by conventional plant breeding techniques. The introduction and expression of the structural gene for phaseolin, for example, can be used to enhance the protein content and nutritional value of forage crops such as alfalfa. Other uses of the invention, exploiting the properties of other structural genes introduced into other plant species will be readily apparent to those skilled in the art. The invention in principle applies to any introduction of a plant structural gene into any plant species into which T-DNA can be introduced and in which T-DNA can remain stably replicated. In general these species include, but are not limited to, dicotyledenous plants, such as sunflower (family *Compositeae),* tobacco (family *Solanaceae),* alfalfa, soybeans and other legumes (family *Leguminoseae)* and most vegetables.

Detailed description of the invention

The following definitions are provided, in order to remove ambiguities to the intent or scope of their usage in the specification and claims.

*T-DNA:* A segment of DNA derived from the tumor-inducing principle (TIP) which becomes integrated in the plant genome. As used herein, the term includes DNA originally derived from any tumor-inducing strain of *Agrobacterium* including *A. tumefaciens* and *A. Rhizogenes,* the inserted segment of the latter sometimes referred to in the prior art as R-DNA. In addition, as used herein the term T-DNA includes any alterations, modifications, mutations, insertions and deletions either naturally occurring or introduced by laboratory procedures, a principle structural requirement and limitation to such modifications being that sufficient right an left ends of naturally-occurring T-DNAs be present to insure the expected function of stable integration in the transformed plant cell genome which is characteristic of T-DNA. In addition, the T-DNA must contain at least one T-DNA promoter in sufficiently complete form to control initiation of transcription and initiation of translation of an inserted plant structural gene. Preferably, an insertion site will be provided "downstream" in the direction of transcription and translation initiated by the promoter, so located with respect to the promoter to enable a plant structural gene inserted therein to be expressed under control of the promoter, either directly or as a fusion protein.

*Plant structural gene:* As used herein includes that portion of a plant gene comprising a DNA segment coding for a plant protein, polypeptide or portion thereof but lacking those functional elements of a plant gene that regulate initiation of transcription and initiation of translation, commonly referred to as the promoter region. A plant structural gene may contain one or more introns or it may constitute an uninterrupted coding sequence. A plant structural gene may be derived in whole or in part from plant genomic DNA, cDNA and chemically synthesized DNA. It is further contemplated that a plant structural gene could include modifications in either the coding segments or the introns which could affect the chemical structure of the expression product, the rate of expression or the manner of expression control. Such modifications could include, but are not limited to, mutations, insertions, deletions, and "silent" modifications that do not alter the chemical structure of the expressin product but which affect intercellular localization, transport, excretion or stability of the expression product. The structural gene may be a composite of segments derived from a plurality of sources, naturally occurring or synthetic, coding for a composite protein, the composite protein being in part a plant protein.

*T-DNA promoter:* Refers to any of the naturally occurring promoters commonly associated with integrated T-DNA. These include, but are not limited to, promoters of the octopine synthetase gene, nopaline synthetase gene, *tms, tml* and *tmr* genes, depending in part on the TIP source of the T-DNA. Expression under control of a T-DNA promoter may take the form of direct expression in which the structural gene normally controlled by the promoter is removed and replaced by the inserted plant structural gene, a start codon being provided either as a remnant of the T-DNA structural gene or as part of the inserted plant structural gene, or by fusion protein expression in which part or all of the plant structural gene is inserted in correct reading frame phase within the existing T-DNA structural gene. In the latter case, the expression product is referred to as a fusion protein.

*Plant tissue:* Includes differentiated and undifferentiated tissues of plants including roots, shoots, pollen, seeds, tumor tissue, such as crown galls, and various forms of aggregations of plant cells in culture, such as embryos and calluses.

*Plant cell:* As used herein includes plant cells *in planta* and plant cells and protoplasts in culture.

Production of a genetically modified plant expressing a plant structural gene introduced via T-DNA combines the specific teachings of the present disclosure with a variety of techniques and expedients known in the art. In most instances, alternative expedients exist for each stage of the overall process. The choice of expedients depends on variables such as the choice of the basic TIP, the plant species to be modified and the desired regeneration strategy, all of which present alternative process steps which those of ordinary skill are able to select and use to achieve a desired result. The fundamental aspects of the invention are the nature and structure of the plant structural gene and its means of insertion into T-DNA. The remaining steps in obtaining a genetically modified plant include transferring the modified T-DNA to a plant cell wherein the modified T-DNA becomes stably integrated as part of the plant cell genome, techniques for *in vitro* culture and eventual regeneration into whole plants, which may include steps for selecting and detecting transformed plant cells and steps of transferring the introduced gene from the originally transformed strain into commercially acceptable cultivars.

A principal feature of the present invention is the construction of T-DNA having an inserted plant structural gene under control of a T-DNA promoter, as these terms have been defined, *supra.* The plant structural gene must be inserted in correct position and orientation with respect to the T-DNA promoter. Position has two aspects. The first relates to on which side of the promoter the structural gene is inserted. It is known that the majority of promoters control initiation of transcription and translation in one direction only along the DNA. The region of DNA lying under promoter control is said to lie "downstream" or alternatively "behind" the promoter. Therefore, to be controlled by the promoter, the correct position of plant structural gene insertion must be "downstream" from the promoter. (It is recognized that a few known promoters exert bi-directional control, in which case either side of the promoter could be considered to be "downstream" therefrom). The second aspect of position refers to the distance, in base pairs, between known functional elements of the promoter, for example the transcription initiation site, and the translational start site of the structural gene. Substantial variation appears to exist with regard to this distance, from promoter to promoter. Therefore, the structural requirements in this regard are best described in functional terms. As a first approximation, reasonable operability can be obtained when the distance between the promoter and the inserted structural gene is similar to the distance between the promoter and the T-DNA gene it normally controls. Orientation refers to the directionality of the structural gene. By convention, that portion of a structural gene which ultimately codes for the amino terminus of the plant protein is termed the 5' end of the structural gene, while that end which codes for amino acids near the carboxyl end of the protein is termed the 3' end of the structural gene. Correct orientation of the plant structural gene is with the 5' end thereof proximal to the T-DNA promoter. An additional requirement in the case of constructions leading to fusion protein expression is that the insertion of the plant structural gene into the T-DNA structural gene sequence must be such that the coding sequences of the two genes are in the same reading frame phase, a structural requirement which is well understood in the art. An exception to this requirement, of relevance to the present invention, exists in the case where an intron separates the T-DNA gene from the first coding segment of the plant structural gene. In that case, the intron splice sites must be so positioned that the correct reading frame for the T-DNA gene and the plant structural gene are restored in phase after the intron is removed by post-transcriptional processing. The source of T-DNA may be any of the TIP plasmids. The plant structural gene is inserted by standard techniques well known to those skilled in the art. Differences in rates of expression may be observed when a given plant structural gene is inserted under control of different T-DNA promoters. Different properties, including such properties as stability, inter-cellular localization, excretion, antigenicity and other functional properties of the expressed protein itself may be observed in the case of fusion proteins depending upon the insertion site, the length and properties of the segment of T-DNA protein included within the fusion protein and mutual interactions between the components of the fusion protein that effect folded configuration thereof, all of which present numerous opportunities to manipulate and control the functional properties of the expression product, depending upon the desired end use. Expression of the phaseolin structural gene has been observed when that gene was inserted under control of the octopine synthetase promoter from an octopine plasmid of *A. tumefaciens.*

A convenient means for inserting a plant structural gene into T-DNA involves the use of a shuttle vector, as described *supra,* having a segment of T-DNA (that segment into which insertion is desired) incorporated into a plasmid capable of replicating in E. *coli.* The T-DNA segment contains a restriction site, preferably one which is unique to the shuttle vector. The plant structural gene can be inserted at the unique site in the T-DNA segment and the shuttle vector is transferred into cells of the appropriate *Agrobacterium* strain, preferably one whose T-DNA is homologous with the T-DNA segment of the shuttle vector. The transformed *Agrobacterium* strain is grown under conditions which permit selection of a double-homologous recombination event which results in replacement of a pre-existing segment of the Ti plasmid with a segment of T-DNA of the shuttle vector.

Following the strategy just described, the modified T-DNA can be transferred to plant cells by any technique known in the art. For example, this transfer is most conveniently accomplished either by direct infection of plants with the novel *Agrobacterium* strain containing a plant structural gene incorporated within its T-DNA, or by co-cultivation of the *Agrobacterium* strain with plant cells. The former technique, direct infection, results in due course in the appearance of a tumor mass or crown gall at the site of infection. Crown gall cells can be subsequently grown in culture and, under appropriate circumstances known to those of ordinary skill in the art, regenerated into whole plants that contain the inserted T-DNA segment. Using the method of co-cultivation, a certain proportion of the plant cells are transformed, that is to say have T-DNA transferred therein and inserted in the plant cell genome. In either case, the transformed cells must be selected or screened to distinguish them from untransformed cells. Selection is most readily accomplished by providing a selectable marker incorporated into the T-DNA in addition to the plant structural gene. Examples include either dihydrofolate reductase or neomycin phosphotransferase expressed under control of a nopaline synthetase promoter. These markers are selected by growth in medium containing methotrexate or kanamycin, respectively, or their analogs. In addition, the T-DNA provides endogenous markers such as the gene or genes controlling hormone-independent growth of Ti-induced tumors in culture, the gene or genes controlling abnormal morphology of Ri-induced tumor roots, and genes that control resistance to toxic compounds such as amino acid analogs, such resistance being provided by an opine synthetase. Screening methods well known to those skilled in the an include assays for opine production, specific hybridization to characteristic RNA or T-DNA sequences, or immunological assays for specific proteins, including ELISA (acronym for "Enzyme Linked Immunosorbant Assay"), radioimmune assays and "western" blots.

An alternative to the shuttle vector strategy involves the use of plasmids comprising T-DNA or modified T-DNA, into which a plant structural gene is inserted, said plasmids being capable of independent replication in an *Agrobacterium* strain. Recent evidence indicates that the T-DNA of such plasmids can be transferred from an *Agrobacterium* strain to a plant cell provided the *Agrobacterium* strain contains certain trans-acting genes whose function is to promote the transfer of T-DNA to a plant cell. Plasmids that contain T-DNA and are able to replicate independently in an *Agrobacterium* strain are herein termed "sub-TIP" plasmids. A spectrum of variations is possible in which the sub-TIP plasmids differ in the amount of T-DNA they contain. One end of the spectrum retains all of the T-DNA from the TIP plasmid, and is sometimes termed a "mini-TIP" plasmid. At the other end of the spectrum, all but the minimum amount of DNA surrounding the T-DNA border is deleted, the remaining portions being the minimum necessary to be transferrable and integratable in the host cell. Such plasmids are termed "micro-TIP". Sub-TIP plasmids are advantageous in that they are small and relatively easy to manipulate directly. After the desired structural gene has been inserted, they can easily be introduced directly into an *Agrobacterium* cell containing the trans-acting genes that promote T-DNA transfer. Introduction into an *Agrobacterium* strain is conveniently accomplished either by transformation of the *Agrobacterium* strain or by conjugal transfer from a donor bacterial cell, the techniques for which are well known to those of ordinary skill.

Regeneration is accomplished by resort to known techniques. An object of the regeneration step is to obtain a whole plant that grows and reproduces normally but which retains integrated T-DNA. The techniques of regeneration vary somewhat according to principles known in the art. depending upon the origin of the T-DNA, the nature of any modifications thereto and the species of the transformed plant. Plant cells transformed by an Ri-type T-DNA are readily regenerated, using techniques well known to those of ordinary skill, without undue experimentation. Plant cells transformed by Ti-type T-DNA can be regenerated, in some instances, by the proper manipulation of hormone levels in culture. Preferably, however, the Ti-transformed tissue is most easily regenerated if the T-DNA has been mutated in one or both of the *tmr* and *tms* genes. Inactivation of these genes returns the hormone balance in the transformed tissue towards normal and greatly expands the ease and manipulation of the tissue's hormone levels in culture, leading to a plant with a more normal hormone physiology that is readily regenerated. In some instances, tumor cells are able to regenerate shoots which carry integrated T-DNA and express T-DNA genes, such as nopaline synthetase, and which also express an inserted plant structural gene. The shoots can be maintained vegetatively by grafting to rooted plants and can develop fertile flowers. The shoots thus serve as parental plant material for normal progeny plants carrying T-DNA and expressing the plant structural gene inserted therein.

## Examples

The following Examples utilize many techniques well known and accessible to those skilled in the arts of molecular biology and manipulation of TIPs and *Agrobacterium;* such methods are not always described in detail. Enzymes are obtained from commercial sources and are used according to the vendor's recommendations or other variations known to the art. Reagents, buffers and culture conditions are also known to those in the art. Reference works containing such standard techniques include the following: R. Wu, ed. (1979) Meth. Enzymol. 68; J. H. Miller (1972) *Experiments in Molecular Genetics;* R. Davis *et al.* (1980) *Advanced Bacterial Genetics;* and R. F. Schleif and P. C. Wnesink (1982) *Practical Methods in Molecular Biology.*

In the Examples, special symbols are used to clarify sequences. Sequences that do or could code for proteins are underlined, and codons are separated with slashes (/). The positions of cuts or gaps in each strand caused by restriction endonucleases or otherwise are indicated by the placement of asterisks (*). (In Example 4 a double-stranded DNA molecule is represented by a single line flanked by asterisks at the sites of restriction enzyme cuts; the approximate position of a gene is there indicated by underlined "X"'s under the single line). With the exception of the plasmid IIc, plasmids, and only plasmids, are prefaced with a "p", e.g., p3.8 or pKS4. Cells containing plasmids are indicated by identifying the cell and parenthetically indicating the plasmid, e.g., *A. tumefaciens* (pTi15955) or K802(pKS4-KB). Table 1 provides an index useful for identifying plasmids and their interrelationships. Table 2 provides an index of deposited strains.

Fig. 28 provides a useful comparison of the constructions described in Examples 4, 5, and 7. Fig. 29 sets forth the genetic code and is useful for interpreting sequences. The nucleotide sequence of an important T-DNA gene, *tml,* though not used in these Examples. is set forth in Fig. 30; it is useful in designing constructions not described herein.

### Example 1

The purpose of this construction is to teach how to construct a Shuttle Vector to be used in pTi system for expressing foreign genes in crown gall cells, the foreign gene being under control of the *nos* promoter, part of which is chemically synthesized, and is missing codons for the nopaline synthetase gene. Prior to the start of construction, a clone of pTiC58 T-DNA (pCF44A) was sequenced to discover the *nos* promoter (Fig. 2).

1.1 Isolation of the 5' portion of the *nos* promoter

pCF44A is cut with *XhoI*, religated, and labelled pCF44B, which has the following structure:

```
    BglII            ClaI          ClaI          SstII         SstII         SstII             BglII
      *   1160 bp     *    1300     *     355     *     620     *     420     *     1155 bp      *
...___________________________________________________________________________________________________...
      *               *XXXXXXXXX*XXXXXXXX*              *             *                         *
                       3'       nopaline    5'
                               synthetase
```

This new plasmid is of the *SstII* fragments. The resulting plasmid, pCF44C

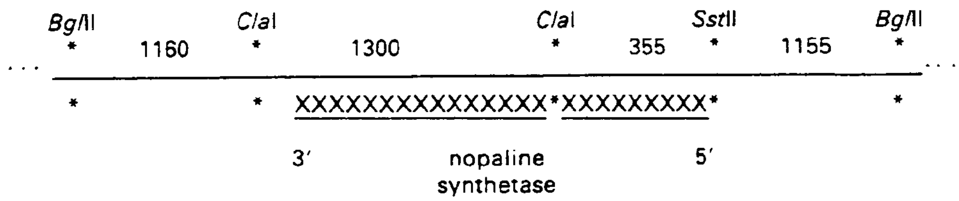


is digested with *Bgl*II, and a 3.6 kbp fragment is inserted into the *Bgl*II site of pRK290. A colony selected for hybridization to T-DNA in a Grunstein-Hogness assay is labelled pKS-nopV, digested with *Cla*I, and religated, forming pKS-nopVI.

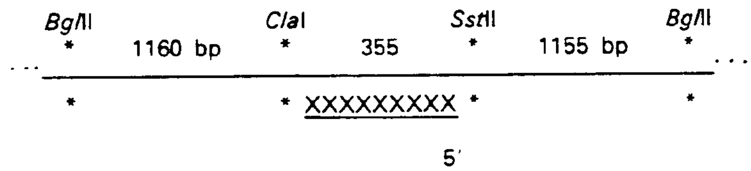


This is digested with *Cla*I and *Sst*II giving a 22 kbp linearized vehicle and a 355 bp fragment. These are easily separated by centrifugation through a salt gradient. After the small fragment is digested with *Hin*fI the 149 bp *Sst*II/*Hin*fI and the 208 bp *Cla*I/*Hin*fI fragments are isolated by gel electrophoresis.

1.2 Synthesis of linkers

The following two linkers were synthesized by the method of Example 10:

a)

5′ AGTCTCATACTCACTCTCAATCCAAATAATCTGCCATGGAT 3′

b)

5′ CGATCCATGGCAGATTATTTGGATTGAGAGTGAGTATGAG 3′

They were annealed together to form the following structure:

5′ AGTCTCATACTCACTCTCAATCCAAATAATCTGCCATGGAT 3′ (a
3′ GAGTATGAGTGAGAGTTAGGTTTATTAGACGGTACCTAGC 5′ (b

This sequence has a *Hin*fI site on the left. and *Nco*I and *Cla*I sites on the right. An alternate sequence will have a *Bcl*I site between the *Nca*I and *Cla*I sites. The sequence is identical to that found in T-DNA except for the underlined bases which replace an A-T base pair with a C-G base pair.

1.3 Assembly of pNNN2

The 22 kbp *Cla*I/*Sst*II vehicle is ligated as shown in Fig. 4 with the 149 bp *Sst*II/*Hin*fI fragment and the synthetic linker, forming the following structure:

*Hin*fI synthetic linker *Nco*I *Cla*I
5′ . . . 149bp . . . TAG*AGT CTCATACTCACTCTCAATCCAAATAATCTGC*CATG GAT*CG AT . . . 1160 bp . . . 3′
3′ . . . T-DNA . . . ATC TCA*GAGTATGAGTGAGAGTTAGGTTTATTAGACG GTAC*CTA GC*TA . . . T- DNA . . . 5′

1.4 Insertion and expression of a phaseolin gene

pNNN2, the plasmid constructed in Example 1.3 (Fig. 4) is cut with *Cla*I, mixed with the *Cla*I/*Eco*RI linker synthesized in Example 1.2 and electrophoretically purified *Eco*RI/*Cla*I *kan*/bean fragment from pKS4-KB, ligated, transformed, isolated, and restriction mapped. The appropriate plasmid, pNNN4, is transferred and tested for expression as described in Example 14, 12 and 13.

Example 2

The purpose of this construction is to ligate the phaseolin gene from the *Eco*RI site to *Bam*HI site, into the active T-DNA gene that lies across the *Hin*dIII sites on p403. The mRNA of this T-DNA gene is labelled 1.6 on the map, shown in Fig. 1, and 1450 bp and Prol in the map shown in Fig 6. This T-DNA gene is referred to herein as the "1.6 transcript" gene. The sequence (see Fig. 5) was determined from the *Hin*dIII site of p401 past the *Cla*I site to its right (see Fig. 9. There is an open reading frame that starts between the *Hin*dIII and *Cla*I site going toward the *Hin*dIII site (see the 1450 bp mRNA mapped in Fig. 6). The *Cla*I site is in the untranslated leader of the mRNA of the gene spanning the *Hin*dIIII sites. We create a promoter vehicle by cutting out the ClaI fragment in the middle of p403. This is possible because the internal *Cla*I sites are not methylated in some *E. coli* strains, whereas the *Cla*I site next to the *Eco*RI site is methylated.

The phaseolin gene is now ligated into the *Cla*I site bringing with it an ATG. This can be accomplished by using pKS4-3.0 KB. The base sequence from the *Cla*I site of pBR322 through the *Eco*RI site of phaseolin is as follows:

```
        ClaI                                                   EcoRI
5'...AT*C /G AT /GAT /AAG/CTG /CTG /TCA /AAC /ATG /AG*A/ATT /CTT /TTC...3'

3'...TA G C*TA CTA TTC GAC GAC AGT TTG TAC TC T  TAA*GAA AAC...5'
                                      Met Arg / Ile  Leu  Phe...
                                                13   14   15

                      ...derived from pBR322/phaseolin...
```

Note the open reading frame and the ATG. There are 18 bp between the *Cla*I site and the translational start signal (ATG). This compares to 12 bp from the *Cla*I site to the start of the T-DNA gene:

```
         ClaI
5'...AT*CG A/TGG/ACA/TGC/TGT/ATG..3'

3'...TA GC*TACC TGT ACG ACA TAC...5'
                            Met...
```

Again, note the open reading frame and the ATG. Thus, ligation into the *Cla*I site of the promoter clone should create an active phaseolin gene in T-DNA. The phaseolin gene has a substitution of 2 amino acids for the naturally occurring amino terminal 12 residues.

2.1 Construction of a promoter vehicle

pKSIII, which is a pRK290 clone corresponding to the T-DNA clone p403 (see Fig. 1), is digested with *Cla*I and then religated. The ligation mix is transformed into K802 and selected for kanamycin resistance. Plasmids are isolated by doing "minipreps" (plasmid preparations from small volume cell cultures) and restriction maps are obtained to prove the structure. The new vehicle, pKS-prol, is not able to be digested by *Hin*dIII but can be linearized by *Cla*I (Fig. 7). pKS-prol is purified and linear molecules are produced by digestion with *Cla*I.

2.2 Ligation of a partial phaseolin gene to a kanamycin resistance gene

A 3.0 kbp fragment containing extensive 3' flanking sequences and all but the extreme 5' coding sequences of the phaseolin gene was obtained by elution from an agarose gel after electrophoresis of an *Hin*dIII and *Bam*HI digest of p7.2 (Fig. 8), a pBR322 subclone of the phaseolin genomic clone 177.4 whose construction is described in Example 3.1. This was mixed with and ligated to a 3.0 kbp kanamycin resistance *Hin*dIII/*Bam*HI fragment similarly isolated from pKS4 (Fig. 13), and *Hin*dIII-linearized pBR322. After restriction mapping of plasmids isolated from ampicillin resistant transformants, a plasmid having the structure shown in Fig. 3 was labelled pKS4-KB.

2.3 Purification of the *kan*/bean fragment from pKS4-3.0KB

pKS4-KB (Fig. 3) is digested with *Cla*I and the 4.9 kbp fragment purified by agarose gel electrophoresis.

2.4 Ligation of *Cla*I *kan*/bean resistance gene into *Cla*I digested pKS-Prol

pKS-prol is linearized by digestion with *Cla*I and the kanamycin resistance gene/bean fragment from Example 5.3 are ligated together and transformed into K802. Kanamycin resistant transformants are selected and plasmids isolated bv "minipreps" are restriction mapped to detect one havina the proper orientation. The plasmid is labelled pKS-prol-KB (Fig. 9).

2.5 Transformation and expression

Cells containing pKS-prol-KB are mated with *Agrobacterium* cells containing pTi15955 or pTiA66 or other appropriate TIP plasmids. After selection of recombinants with kanamycin, plants are inoculated and crown qalls are established in tissue culture. Testinq for the svnthesis of phaseolin is as described in Examples 12 and 13.

Example 3

This example teaches manipulations of a gene for phaseolin, the major seed storage protein of the bean *Phaseolus vulgaris* L., preparatory to further manipulations which insert the phaseolin gene into vectors described in various other examples.

3.1 Subcloning of a phaseolin gene

A genomic clone of phaseolin in a Charon 24A AG-PVPh177.4 (or 177.4; S. M. Sun *et al.* (1981) Nature *289*:37-41, J. L. Slightom et *al.* (1983) Proc. Natl. Acad. Sci. USA *80*; Fig 10) was digested with *Bgl*II and *Bam*HI. The 3.8 kbp fragment carrying the phaseolin gene and its flanking sequences, isolated by agarose gel electrophoresis, was mixed with and ligated to *Bam*HI-linearized pBR322. The mixture was transformed into HB101, and colonies resistant to ampicillin and sensitive to tetracycline were selected. Plasmid isolated from these clones was restriction mapped. A plasmid having the structure shown in Fig. 11 was selected and labelled AG-pPVPh3.8 (or alternatively, p3.8). The ligation of *Bgl*II and *Bam*HI sites with each other inactivates both sites.

Another subclone of 177.4 was constructed by digestion with *Eco*RI, isolation of a 7.2 kbp fragment containing extensive 3' flanking sequences and all but the extreme 5' end of the phaseolin gene, and isolated after ampicillin selection of HB101 transformants were restriction mapped. A plasmid having the insert oriented so that the *Hin*dIII site of pBR322 was adjacent to the 5' end of the phaseolin gene and distal to the 3' untranslated region was labelled AG-pPVPh7.2 (or p7.2; Fig. 8; Sun *et al.* and Slightom *et al.*, *supra).*

3.2 Cloning and isolation of a kanamycin resistance gene

pRZ102 (R. A. Jorgenson *et al.* (1979) Molec. gen. Genet. 177:65-72), a ColE1 plasmid carrying a copy of the transposon Tn5, was digested with *BamH*I and *Hin*dIII, mixed with pBR322 (Fig. 12) previously linearized with the same two enzymes, ligated, and transformed into K802. Plasmids, isolated from transformants selected for resistance to both ampicillin and kanamycin were restriction mapped and one having the structure shown in Fig. 13 was labelled pKS-4.

3.3 Linkage of the phaseolin gene with a kanamycin resistance gene

p3.8 was digested with *Cla*I and *Bam*HI, and a 4.2 kbp fragment containing the phaseolin gene and some pBR322 sequences was isolated by agarose gel electrophoresis. This was mixed with a *Cla*I/*Bam*HI fragment of Tn5 carrying a kanamycin resistance (neomycin phosphotransferase II) gene from pKS4 (Fig. 13) and pBR322 (Fig. 12) which had been linearized with *Cla*I. The mixture was ligated and transformed into K802. After selection of colonies resistant to ampicillin and kanamvcin, plasmids were isolated and restriction mapped. A colony having the structure shown in Fig. 14 was labelled pKS-KB3.8.

The construction of another useful plasmid, pKS4-KB, is described in Example 2.2.

Example 4

The purpose of this example is to generate a Ti plasmid with a deletion from the *tms* ("shooting" locus) through the *tmr* ("rooting" locus) of pTi15955 and other octopine Ti plasmids. This derivative is useful because cells transformed by it are easier to regenerate to whole plants than cells transformed by pTi15955 with intact *tms* and *tmr* genes.

The *tms-tmr* deleted pTi15955 is ultimately changed in two ways: the inactivation of *tms-tmr* and the insertion of a foreign gene. Should these two changes be located at different points of the T-DNA, each change is inserted independently by different shuttle vectors. Each shuttle vector dependent change is selected independently which will necessitate use of at least two markers selectable in *Agrobacterium.* In addition to the usual kanamycin resistance, this example utilized a chloramphenicol resistance derived from pBR325.

4.1 Construction of a chloramphenicol resistance gene clone

pBR325 is digested with *Hinc*II and blunt end ligated with *Hin*dIII linkers. The resultant preparation is digested with *Hin*dIII, religated, selected for chloramphenicol resistance *(cam),* and labelled pKS-5 which will serve as a source of the *Hin*dIII/*Bcl*I fragment which contains the *cam* gene (Fig. 15).

4.2 Construction of a pBR322 clone of T-DNA with a deletion and a *cam* gene

A 9.2 kbp linear DNA fragment is isolated from a complete *Hin*dIII and partial *Bam*HI digest of p203. The fragment carrying the *cam* gene is isolated from pKS-5, mixed with the 9.2 kbp linear fragment, ligated, transformed into *E. coli,* selected for chloramphenicol resistance. and labelled pKS-Oct.Cam203 (Fig. 16).

pKS-Oct.Cam203 is a plasmid clone that can now be used to construct a number of deletion TL mutants of pTi15955. It contains the right hand arm of TL and a resistance gene to the left of the right arm. We can attach various left-hand arms of TL to the left of the *cam* gene (*Hin*dIII site). For instance, if p102 is attached the deletion is 5.2 kbp long and includes all of *tms* and tmr. If p103 is attached the deletion is 3.2 kbp long and includes part of *tms* and all of *tmr.* See Fig. 1.

pKS-Oct.Cam203 is digested with *Hin*dIII. p102 or p103 is digested with *Hin*dIII and the 2.2 kbp or 2.0 kbp T-DNA fragment is isolated and ligated with the linearized pKS-Oct.Cam203, transformed, isolated yielding pKS-Oct.delII (Fig. 17) or pKS-Oct.delI (Fig. 18), respectively. These constructions are moved into *A. tumefaciens* by mating, homologous recombinations, and selection for chloramphenicol resistance. Alternatively, one moves the constructions into pRK290 by use of established methods by linearizing the construction carrying plasmids with *Bam*HI and ligating into the *Bgl*II site of pRK290 (Fig. 19).

Example 5

The Ti plasmid is mutated in this example by deleting the T-DNA between the *Hpa*I site in *tmr* to the *Sma*I site in *tml*. The Ti plasmids that can be modified include pTi15955, pTiB6, pTiA66 and others. This construction is diagrammed in Fig. 20.

5.1 Isolation of the *cam* gene

pKS-5 (Fig. 15) is digested with *Hin*dIII and *Bcl*I. The smallest fragment is isolated after separation on an agarose gel, as taught in Example 4.

5.2 Construction of a pBR322 clone of T-DNA with a deletion

The right hand arm of the T-DNA deletion is constructed by insertion of *Bgl*II sites into the *Sma*I sites of p203 (see Fig. 1). p203 is digested by *Sma*I, ligated with *Bgl*II linkers, digested with *Bgl*II, religated, and transformed into K802. In an alternative construction, *Bam*HI linkers may be substituted for *Bgl*II linkers and the appropriate *Bam*HI partial digest products are isolated). The resultant plasmid is labelled p203-*Bgl*II, and is digested with *Bgl*II and *Hin*dIII. The large *Bgl*II/*Hin*dIII vector containing fragment is ligated with the chloramphenicol resistance fragment whose isolation was described in Example 5.1. Chloramphenicol resistance is selected for after transformation into K802. The resultant plasmid is labelled p2f (Fig. 20).

5.3 Construction are left-hand arm of T-DNA deletion clone

*Hind*III sites are inserted into the *Hpa*I site of p202 by digestion with *Hp*aI and ligation with *Hin*dIII linkers. After unmasking of the *Hin*dIII sticky ends by digestion with that restriction enzyme, the 2 kbp *Hpa*I fragment which now bears *Hin*dIII ends is isolated. The *Hin*dIII digested *Hin*dIII-ended *Hpa*I fragment is transformed into K802. After a colony containing the desired construction is isolated, and characterized, the plasmid is labelled p3e (Fig. 21).

5.4 Construction of the T-DNA deletion clone

The left-hand arm of the clone is obtained by purifying a 2 kbp fragment of a *Hin*dIII digest of p3e by elution from an agarose gel after electrophoresis. p2f is cut by *Hin*dIII, treated with alkaline phosphatase, mixed with the 2 kbp fragment, ligated, transformed into K802, and selected for chloramphenicol resistance. Plasmids are isolated from individual colonies and characterized by restriction mapping. A plasmid having the two arms in the desired tandem orientation is chosen and labelled pKS-Oct.delIII (Fig. 22).

pKS-Oct.delIII is moved into *A. tumefaciens* by mating, and homologous recombinants are selected with chloramphenicol. Sunflower and tobacco roots and shoots are inoculated as described in other Examples and the tumors generated are tested for opines.

Example 6

This example teaches a construction deleting *tmr* and *tml* that provides an alternative to that taught in Example 5.

6.1 Construction of a chloramphenicol resistant fragment with a *Bgl*II site

pBR325 is digested with *Hinc*II, blunt-end ligated with *Bgl*II linkers, digested with *Bgl*II, and religated (Fig. 23). Chloramphenicol resistance is selected for after transformation of either K802 or GM33. The resultant plasmid, pKS-6 serves as a source of the *Bgl*II/*Bcl*I fragment carrying the *cam* gene.

6.2 Construction of the *tmr, tml* deletion clone

p203 is digested with *Hpa*I and *Sma*I. After blunt end ligation with *Bgl*II linkers, it is digested with *Bgl*II to expose the *Bgl*II sticky-ends, religated, and transformed into K802. The desired construction is identified and labelled p2 (Fig. 24).

6.3 Construction of the T-DNA deletion clone (pKS-Oct.delIIIa)

The *Bgl*II fragment carrying the *cam* gene is isolated from pKS-6 and ligated into *Bgl*II-cut p2. Chloramphenicol resistance is selected for after transformation of K802. The resultant plasmid is labelled pKS-Oct.delIIIa (Fig. 25), and is tested as described in Example 5.4.

Example 7

The purpose of this construction is to provide an example of the mutation of the *tmr* locus only at the *Hpa*I site by insertion of the chloramphenicol resistance gene. This gene is isolated as the *Bgl*II/*Bcl*I fragment from pKS-6, and is ligated into the *Hpa*I site of p203 after that site is changed to a *Bgl*II site.

7.1 Conversion of the *Hpa*I site to a *Bgl*II site

p203 is digested with *Hpa*I, ligated to *Bgl*II linkers, trimmed with *Bgl*II and religated. After transformation of K802, colonies are selected and screened by restriction mapping for insertion of *Bgl*II sites (Fig.26).

7.2 Isolation of the *cam* gene

pKS-6 is digested with *Bgl*II and *Bcl*I. The smallest fragment is isolated by agarose gel electrophoresis.

7.3 Construction of the mutated T-DNA clone

The modified p203 from Example 7.1 is digested with *Bgl*II, ligated with the purified *cam* gene from Example 7.2 and transformed into K802. Chloramphenicol resistance is selected for, and after isolation from the resistant transformants and characterization by restriction enzyme mapping, the plasmid is labelled pKS-Oct.*tmr* (Fig. 27).

Example 8

Regeneration in this example involves carrot tumors incited by an Ri-based TIP plasmid and is effected essentially as described by M. D. Chilton *et al.* (1982) Nature *295*:432-434.

8.1 Infection with hairy root

Carrot disks are inoculated with about 10594 bacteria in 0.1 ml of water. One to 1.5 cm segments of the ends of the roots obtained are cut off, placed on solid (1-1.5% agar) Monier medium lacking hormones (D. A. Tepfer and J. C. Tempe (1981) C. R. Hebd. Seanc. Acad. Sci., Paris *295*:153-156). and grown at 25°C to 27°C in the dark. Cultures uncontaminated by bacteria are transferred every 2 to 3 weeks and are subcultured in Monier medium lacking hormones and agar.

8.2 Regeneration of roots to plants

The cultured root tissue described in Example 8.1 is placed on solidified (0.8% agar) Monier medium supplemented with 0.36 μM 2,4-D and 0.72 μM kinetin. After 4 weeks, the resulting callus tissue is placed in liquid Monier medium lacking hormones. During incubation at 22 to 25°C on a shaker (150 r.p.m.) for one month, the callus disassociates into a suspension culture from which embryos differentiate, which, when placed in Petri dishes containing Monier medium lacking hormone, develop into plantlets. These plantlets are grown in culture. and after "hardening" by exposure to atmospheres of progressively decreasing humidity, are transferred to soil in either a greenhouse or field plot.

8.3 Use of non-hairy root vectors

Ti-based vectors which do not have functional *tmr* genes are used instead of the Ri-based vectors as described in Examples 8.1 and 8.2. Construction of suitable deletions is described in Examples 5, 6 and 7.

Example 9

Regeneration in this example involves tobacco tumors incited by a Ti-based TIP plasmid and is effected essentially as described by K. A. Barton *et al.* (1983) Cell, *32*:1033-1034.

9.1 Infection with crown gall

Tobacco tissue is transformed using an approach utilizing inverted stem segments first described by A. C. Braun (1956) Canc. Res. *16*:53-56. Stems are surface sterilized with a solution that was 7% commercial Chlorox and 80% ethanol, rinsed with sterile distilled water, cut into 1 cm segments, and placed basal end up in Petri dishes containing agar-solidified MS medium (T. Murashige and F. Skoog (1962) Physiol. Plant. *15*:473-497) lacking hormones. Inoculation is effected by pucturing the cut basal surface of the stem with a syringe needle and injecting bacteria. Stems are cultured at 25°C with 16 hours of light per day. The calli which develop are removed from the upper surface of the stem segments, are placed on solidified MS medium containing 0.2 mg/ml carbenicillin and lacking hormones, are transferred to fresh MS-carbenicillin medium three times at intervals of about a month, and are tested to ascertain whether the cultures had been ridden of bacteria. The axenic tissues are maintained on solidified MS media lacking supplements under the culture conditions (25°C; 16 hr:8 hr light:dark) described above.

9.2 Culture of transformed tissue

Clones are obtained from the transformed axenic tissues as described by A. Binns and F. Meins (1979) Planta *145*:365-369. Calli are converted into suspensions of cells by culturing in liquid MS having 0.02 mg/l naphthalene acetic acid (NAA) at 25°C for 2 or 3 days while being shaken at 135 r.p.m., and filtering in turn through 543 and 213 μm stainless steel meshes. The passed filtrate is concentrated, plated in 5 ml of MS medium containing 0.5% melted agar, 2.0 mg/l NAA, 0.3 mg/l kinetin and 0.4 g/l Difco yeast extract at a density of about 8 × 10534 cells/ml. Colonies reaching a diameter of about 1 mm are pciked by scalpel point, placed onto and grown on solidified MS medium having 2.0 mg/l NAA and 0.3 mg/l kinetin. The resulting calli are split into pieces and tested for transformed phenotypes.

9.3 Regeneration of plants

Transformed clones are placed onto solidified MS medium having 0.3 mg/l kinetin, and cultured as described in Example 9.1. The shoots which form are rooted by putting them on a solid (1.0% agar) medium containing 1/10 strength MS medium salts, 0.4 mg/l thiamine, lacking sucrose and hormones, and having a pH of 7.0. Rooted plantlets are grown in culture, hardened as described in Example 8.2, and are transferred to soil in either a greenhouse or field plot.

9.4 Vectors used

The methods described in Examples 9.1, 9.2 and 9.3 are suitable Ti-based vectors lacking functional *tmr* genes. Construction of suitable deletions is described in Examples 5, 6 and 7. These methods are also effective when used with Ri-based vectors. The method described in Example 9.1 for infection of inverted stem segments is often useful for the establishment of TIP transformed plant cell lines.

Example 10

The techniques for chemical synthesis of DNA fragments used in these Examples utilize a number of techniques well known to those skilled in the art of DNA synthesis. The modification of nucleosides is described by H. Schaller *et al.* (1963) J. Amer. Chem. Soc. *85*:3821-3827. The preparation of deoxynucleoside phosphoramidites is described by S. L. Beaucage and M. H. Caruthers (1981) Tetrahedron Lett. *22*:1859. Preparation of solid phase resin is described by S. P. Adams *et al.* (1983) J. Amer. Chem. Soc. Hybridization procedures useful for the formation of double-stranded synthetic linkers are described by J. J. Rossi *et al.* (1982) J. Biol. Chem. *257*:9225-9229.

Example 11

Phaseolin is the most abundant storage protein (approximately 50% of the total seed protein) of *Phaseolis vulgaris.* Transfer of the functional phaseolin gene to alfalfa plants and translation of the phaseolin m-RNA into stored phaseolin is of significant economic value since it introduces storage protein into leaf material to be used as fodder. Alfalfa is a valuable plant for the transfer and expression of the phaseolin gene because of its acceptance as cattle fodder, its rapid growth, its ability to fix nitrogen through rhizobial symbiosis, its susceptibility to crown gall infection and the ability to regenerate alfalfa plants from single cells or protoplasts. This example teaches the introduction of an expressible phaseolin gene into intact alfalfa plants.

11.1 Construction of shuttle vector

Alfalfa plants are regenerated from crown gall tissue containing genetically engineered *Agrobacterium* plasmids as described hereafter. In the first step we construct a "shuttle vector" containing a *tmr*5-4 and a *tms*5-T-DNA mutant linked to a phaseolin structural gene under control of a T-DNA promoter. This construction is, in turn, linked to a nopaline synthetase promoter which has a functional neomycin phosphotransferase (NPTII) structural gene (kanamycin resistance) downstream (reported by M. D. Chilton, *et al.* (18 January 1983) 15th Miami Winter Symposium; see also J. L. Marx (1983) Science *219*:830 and R. Horsch *et al.* (18 January 1983) 15th Miami Winter Symposium). A phaseolin structural gene under control of a T-DNA promoter is illustrated in Example 2.

11.2 Transfer to *Agrobacterium* and plant cells

The "shuttle vector" is then transformed by conventional techniques (Example 14) into a strain of *Agrobacterium* containing a Ti plasmid such as pTi15955. Bacteria containing recombinant plasmids are selected and co-cultivated with alfalfa protoplasts which are regenerated cell walls (Marton *et al.* (1979) Nature *277*:129-131; G. J. Wullems *et al.* (1981) Proc. Nat'l Acad. Sci. (USA) *78*:4344-4348; and R. B. Horsch and R. T. Fraley (18 January 1983) 15th Miami Winter Symposium).

Cells are grown in culture and the resulting callus tissue is tested for the presence of the appropriate mRNA by Northern blotting (Example 19) and for the presence of the appropriate proteins by ELISA tests (Example 13) (see J. L. Marx (1983) Science *219*:830; R. B. Horsch and R. T. Fraley (18 January 1983) 15th Miami Winter Symposium).

11.3 Plant regeneration

Alfalfa plants are then regenerated from callus tissue by methods similar to those previously used by A. V. P. Dos Santos *et al.* (1980) Z. Pflanzenphysiol. *99*:261-270. T. J. McCoy and E. T. Bingham (1977) Plant Sci. Letters *10*:59-66 and K. A. Walker *et al.* (1979) Plant Sci. Letters *16*:23-30. These regenerated plants are then propagated by conventional plant breeding techniques forming the basis for new commercial varieties.

Example 12

In all Examples, RNA was extracted, fractionated, and detected by the following procedures.

12.1 RNA extraction

This procedure was a modification of Silflow *et al.* (1981) Biochemistry *13*:2725-2731. Substitution of LiCl precipitation for CsCl centrifugation was described by Murray *et al.* (1981) J. *Mol. Evol.* 17:31-42. Use of 2M LiCl plus 2M urea to precipitate was taken from Rhodes (1975) J. Biol. Chem. *25*:8088-8097.

Tissue was homogenized using a polytron or ground glass homogenizer in 4-5 volumes of cold 50 mM Tris-HCl (pH 8.0) containing 4% p-amino salicylic acid, 1% tri-isopropyl naphthalene sulfonic acid, 10 mM dithiothreitol (freshly made) and 10 mM Na-metabisulfite (freshly made). N-octanol was used as needed to control foaming. An equal volume of Tris-saturated phenol containing 1% 8-hydroxyquinoline was added to the homogenate which was then shaken to emulsify and centrifuged at 20,000-30,000 g for 15 minutes at 4°C. The aqueous upper phase was extracted once with chloroform/octanol (24:1) and centrifuged as above. Concentrated LiCl-urea solution was then added to a final concentration of 2M each and the mixture was left to stand at 20°C for several hours. The RNA precipitate was then centrifuged down and washed wth 2M LiCl to disperse the pellet. The precipitate was then washed with 70% ethanol-0.3M Na-acetate and dissolved in sufficient sterile water to give a clear solution. One half volume of ethanol was added and the mixture put on ice for 1 hour, after which it was centrifuged to remove miscellaneous polysaccharides. The RNA precipitate was then recovered and re-dissolved in water or in sterile no salt poly(U) buffer.

12.2 Poly(U)/Sephadex chromatography

Two poly(U) Sephadex (trademark: Pharmacia, Inc., Uppsala, Sweden) buffers were used; the first with no salt containing 20 mM Tris, 1 mM EDTA and 0.1% SDS, and the second with 0.1M NaCl added to the first. In order to obtain a good match at A42605, a 2× stock buffer should be made and the salt added to a portion. After adjusting the final concentrations, the buffers were autoclaved.

Poly(U) Sephadex was obtained from Bethesda Research Laboratories and 1 gm poly(U) Sephadex was used per 100 μg expected poly(A)RNA. The poly(U) Sephadex was hydrated in no salt poly-U buffer and poured into a jacketed column. The temperature was raised to 60°C and the column was washed with no salt buffer until the baseline at 260 mm was flat. Finally the column was equilibrated with the salt containing poly(U) buffer at 40°C. The RNA at a concentration of less than 500 μg/ml was then heated in no salt buffer at 65°C for 5 minutes, after which it was cooled on ice and NaCl added to a concentration of 0.1M. The RNA was then placed on the column which should be run at no more than 1 ml/min until the optical density has fallen to a steady baseline. The column temperature was then raised to 60°C and the RNA was eluted with no salt poly(U) buffer. The RNA will usually wash off in three column volumes. The eluted RNA was then concentrated with secondary butanol to a convenient volume after addition of NaCl to 10 mM, and precipitated with 2 volumes ethanol. The ethanol precipitate was dissolved in water and NH445-acetate added to 0.1M, followed by re-precipitation with ethanol. Finally the RNA was redissolved in sterile water and stored at -70°C.

12.3 Formaldehyde RNA gels

The method used followed that of Thomas (1980) Proc. Nat'l. Acad. Sci. (USA) 77:5201 and Hoffman, *et al.* (1981) J. Biol. Chem. 256:2597.

0.75-1.5% agarose gels containing 20 mM Na-phosphate (pH 6.6-7.0) were cast. If high molecular weight aggregate bands appeared, then the experiments were repeated with the addition of 6% or 2.2M formaldehyde (use stock solution of 36%) to the gels. The formaldehyde was added to the agarose after cooling to 65°C. Addition of formaldehyde caused visualization with ethidium bromide to be very difficult. The running buffer was 10 mM Na-phosphate (pH 6.8-7.0).

Prior to electrophoresis, the RNA was treated with a denaturing buffer having final concentrations of 6% formaldehyde, 50% formamide, 20 mM Na-phosphate buffer and 5 mM EDTA. The RNA was incubated in the buffer at 60°C for 10-20 minutes. The incubation was terminated by addition of stop buffer. For a 20 µl sample, 4 µl 50% glycerol, 10 mM EDTA, 5 mM Na-phosphate and brompheonl blue were added.

Submerged electrophoresis was used. The RNA was loaded before the gel was submerged, and run into the gel at 125 mA for 5 minutes. The gels were then submerged and the current reduced to 30 mA (overnight) or 50 mA (6-8 hours). The buffer was recirculated and the electrophoresis was done in a cold room.

12.4 "Northern" blots

If the gel was to be blotted to detect a specific RNA, it was not stained; but a separate marker lane was used for staining. Staining was with 5 µg/ml ethidium bromide in 0.1 M Na-acetate and destaining was for several hours in 0.1 M Na-acetate. Treatment in water at 60-70°C for 5-10 minutes prior to staining helped visualization.

A gel to be blotted was soaked for 15 minutes in 10× standard saline citrate (SSC)-3% formaldehyde. If large RNA molecules were not eluting from the gel then a prior treatment in 50 mM NaOH for 10-30 minutes helped to nick the RNA. If base treatment was used, the gel should be neutralized and soaked in SSC-formaldehyde before blotting. Transfer of the RNA to nitrocellulose was done by standard methods.

Prehybridization was done at 42°C for a minimum of 4 hours in 50% formamide, 10% dextran sulfate, 5×SSC, 5×Denhardt's, 100 µg/ml denatured carrier DNA, 20 µg/ml poly(A), 40 mM Na-phosphate (pH 6.8-7.0) and 0.2% SDS. Hybridization was done by addition of the probe to the same buffer with overnight incubation. The probe was not be used at more than approximately 5×10554 c.p.m./ml.

After hybridization, the nitrocellulose was washed a number of times at 42°C with 2×SSC, 25 mM Na-phosphate, 5 mM EDTA and 2 mM Na-pyrophosphate followed by a final wash for 20 minutes at 64°C in 1 ×SSC. Best results were obtained if the filter was not dried prior to autoradiography and the probe could be removed by extensive washing in 1 mM EDTA at 64°C. 13

Example 13

"Western" blots, to detect antigens after SDS-polyacrylamide gel electrophoresis, were done essentially as described by R. P. Legocki and D. P. S. Verma (1981) Analyt. Biochem. *111*:385-392. Micro-ELISA (Enzyme-Linked Immuno-Sorbant Assay) assays were done using immulon-2 type plates with 96 wells by the following steps:

13.1 Binding antibody to plates

On Day 1, the wells were coated with 1:1000 dilution of antibody (rabbit antiphaseolin IgG) in coating buffer. 200 µl/well incubated at 37°C for 2-4 hours. The plates were covered with Saran Wrap. Then the plates were rinsed three times with phosphate buffered saline-Tween (PBS-Tween) allowing a 5 minute waiting period between each rinse step. Then 1 % bovine serum albumin (BSA) was added to rinse and, after addition to the well, left to sit for 20 minutes before discarding. Rinsing was repeated five times more with PBS-Tween.

13.2 Tissue homogenization

The tissue was sliced up into small pieces and then homogenized with a polytron using 1 gm of tissue/ml phosphate buffered saline-Tween-2% polyvinyl pyrrolidone-40 (PBS-Tween-2% PVP40). All samples were kept on ice before and after grinding and standard phaseolin curves were obtained. One standard curve was done in tissue homogenates and one standard curve was also done in buffer to check the recovery of phaseolin when ground in tissue. Following centrifugation of the homogenized samples, 100 µl of each sample were placed in a well and left overnight at 4°C. To avoid errors, duplicates of each sample were done. The plates were sealed during incubation.

13.3 Binding enzyme

After the overnight incubation, the antigen was discarded and the wells were washed five times with PBS-Tween allowing 5 minutes between each rinse.

A conjugate (rabbit anti-phaseolin IgG alkaline phosphatase-linked) was then diluted 1:3000 in PBS-Tween-2% PVP containing 0.2%BSA and 150 µl was added to each well; followed by incubation for 3-6 hours at 37°C. After the incubation, the conjugate was discarded and the wells were rinsed five times with PBS-Tween, allowing five minutes between each rinse as before.

13.4 Assay

Immediately before running the assay, a 5 mg tablet of p-nitrophenyl phosphate (obtained from Sigma and stored frozen in the dark) was added per 10 ml substrate and vortexed until the tablet was dissolved. 200 µl of the room temperature solution was quickly added to each well. The reaction was measured at various times, e.g. t=0, 10, 20, 40, 60, 90 and 120 minutes, using a dynatech micro-elisa reader. When p-nitrophenyl phosphate, which is colorless, was hydrolysed by alkaline phosphatase to inorganic phosphate and p-nitrophenol, the latter compound gave the solution a yellow color, which could be spectrophotometrically read at 410 nm. The lower limit of detection was less than 0.1 ng.

Example 14

Triparental matings were generally accomplished as described below; other variations known to those skilled in the art are also acceptable. *E. coli*K802 (pRK290-based shuttle vector) was mated with *E. coli*(pRK2013) and an *A. tumefaciens* strain resistant to streptomycin. The pRK2013 transferred to the shuttle vector carrying strain and mobilized the shuttle vector for transfer to the *Agrobacterium.* Growth on a medium containing both streptomycin and the drug to which the shuttle vector is resistant, often either kanamycin or chloramphenicol, resulted in the selection of *Agrobacterium* cells containing shuttle vector sequences. A mating of these cells with *E coli*(pPH1J1) resulted in the transfer of pPH1J1 to the Agrobacterium cells. pPH1J1 and pRK290-based shuttle vectors cannot coexist for long in the same cell. Growth on gentamycin, to which pPH1J1 carries a resistance gene, resulted in selection of cells having lost the pRK290 sequences. The only cells resistant to streptomycin, gentamycin, and either kanamycin or chloramphenicol are those which have Ti plasmids that have undergone double-homologous recombination with the shuttle vector and now carry the desired construction.

The strains listed in Table 2 all resemble their parent strains in morphology. Similarly. the physiological characteristics of the listed strains are generally those of the parent strains, except where otherwise indicated in any of the foregoing description. Table 1 and the Figures.

TABLE 1

| Plasmid, bacterium, strain, etc. | Made or used in Example: | Shown in figure: | Made out of | References, comments, or synonyms |
|---|---|---|---|---|
| Charon 24A | 3.1 | | | Bacteriophage vector based on lambda |
| ColEI | 3.2 | | | |
| GM33 | 3.2 | | | *E. coli* |
| HB101 | 3.1 | | | *E. coli* |
| K802 | ubiquitous | | | *E. coli*, W. B. Wood (1966) J. Mol. Biol. *16*:118 |
| pBR322 | | 12 | | F. Bolivar, *et al.* (1978) Gene *2*:95—113 |
| pBR325 | | 23, 15 | | F. Bolivar (1978) Gene *4*:121—136 |
| pcDNA31 | | 10 | pBR322/phas. cDNA | equivalent to pMC6 |
| pCF448 | 1.1 | | pCF44A | |
| pCF44C | 1.1 | | pCF448 | |
| pJS3.8 | | | | =p3.8 |
| pKS-KB3.8 | 3.3 | 19 | pBR322, pKS-4, p3.8 | |
| pKS-nopV | 2.1 | | pCF44c, pRK290 | |
| pKS-nopVI | 2.1 | 4 | pKS-nopV | |

TABLE 1 (continued)

| Plasmid, bacterium, strain, etc. | Made or used in Example: | Shown in figure: | Made out of | References, comments, or synonyms |
|---|---|---|---|---|
| pKS-oct.cam203 | 4.2 | 16 | pKS-5, p203 | =p2f |
| pKS-oct.delI | 4.2 | 28, 18 | pKS-oct.cam203, p103 | |
| pKS-oct.delII | 4.2 | 28, 18 | pKS-oct.cam203, p102 | |
| pKS-oct.delIII | 5.4 | 22 | p2f, p3e | p2f-rt./p3e-ft. |
| pKS-oct.delIIIa | 6.3 | 28, 25 | pKS-6, p2 | |
| pKS-oct.tmr | 7.3 | 28, 27 | pKS-6, p203 | |
| pKS-prol | 2.1 | 7 | pKS111 | |
| pKS-prol-KB | 2.4 | 9 | pKS-prol, pKS4-KB | |
| pKS4 | 3.2 | 13 | pBR322, pRZ102 | |
| pKS4-KB | 2.2 | 3 | pBR322, pKS4, p7.2 | =pKS4-3.0KB, =pKS4-KB3.0 |
| pKS-5 | 4.1 | 15 | pBR325 | |
| pKS-6 | 6.1 | 23 | pBR325 | |
| pKS111 | (2.1) | 7 | pRK290, p403 | |
| pNNN2 | 1.3 | 4 | pKS-nopVI | |
| pNNN4 | 1.4 | | pKS4-KB, pNNN2 | |
| pPH1J1 | 14 | | | used to eliminate shuttle vector, same exclusion group as pRK290, carries gene for resistance to gentamycin, P. R. Hirsh (1973) Thesis, Univ. E. Anglia |

TABLE 1 (continued)

| Plasmid, bacterium, strain, etc. | Made or used in Example | Shown in figure: | Made out of | References, comments, or synonyms |
|---|---|---|---|---|
| pRK290 | common | 19 | | G. Ditta, *et al.* (1980) Proc. Nat. Acad. Sci. USA 77:7347—7357 |
| pRK2013 | 14 | | | used to mobilize the shuttle vector, carries *tra* genes that mobilize a *mob* site on pRK290 for conjugational transfer of pRK290 to *Agrobacterium*, D. H. Figurski & D. R. Helinski, (1979) Proc. Nat. Acad. Sci. USA *76*:1648—1652 |
| pRZ102 | (3.2) | | ColE1, Tn5 | =p7.2 |
| pTiA66 | 2, 5 | | | octopine-type plasmid, pTiA6 with a natural insertion in *tms* pTiB6 |
| pTiB6 | (5) | | | |
| pTiC58 | (1) | | | nopaline-type plasmid |
| pTi15955 | common | 1, 6 | | octopine-type plasmid |
| p2 | 6.2 | 24 | p203 | |
| p2f | 5.2 | 20 | pKS5, p203-BglII | =pKS-oct.cam203 |
| p2f-rt./p3e-1ft. | | 28 | | =pKS-oct.cam203 |
| p2f-rt./p102-1ft. | 5.5 | 28 | p2f, p102 | |
| p2f-rt./p103-1ft. | 5.5 | 28 | p2f, p103 | |
| p3e | 5.3 | 21 | pBR322, p202 | |
| p3.8 | 3.1 | 11 | pBR322, 177.4 | =pJS3.8 |
| p7.2 | 3.1 | 8 | pBR322, 177.4 | =pSS7.2, S. M. Sun, *et al.* (1981) Nature *287*:32—41 |
| p202 | | 21 | pBR322, pTi15955 | |
| p203 | 4, 5, 6, 7 | 20, 24, 25, 31 | pBR322, pTi15955 | |
| p203-BglII | 5.2 | 20 | p203 | |
| p401 | 2 | 6 def, | pBR322, pTi15955 | |

TABLE 1 (continued)

| Plasmid, bacterium, strain, etc. | Made or used in Example: | Shown in figure: | Made out of | References, comments, or synonyms |
|---|---|---|---|---|
| p403 | 2 | | pBR322, pTi15955 | |
| 177.4 | (3.1) | 10 | Charon 24A/phas. gene | AG-PVPh177.4, S. M. Sun *et al.* (1981) Nature *289*:37—41 |

TABLE 2

| | |
|---|---|
| NRRL B-15376 | *A. tumefaciens*/p15955-12A |
| NRRL B-15394 | *E. coli* C600/pKS4 |
| NRRL B-15392 | *E. coli* HB101/p3.8 |
| NRRL B-15391 | *E. coli* HB101/pcDNA31 |
| ATCC 39181 | *E. coli* HB101/pPVL134 |

## Claims

1. A DNA vector comprising T-DNA having a plant structural gene inserted therein under the control of the promoter of the "1.6" transcript of T-DNA, the promoter and the plant structural gene being in such a position and orientation with respect to each other that the plant structural gene is expressible in a plant cell under control of the T-DNA promoter, and the plant structural gene comprising an intron.

2. A DNA vector according to claim 1 wherein the T-DNA contains an inactivating mutation in *tms* or *tmr.*

3. A bacterial strain containing and replicating a vector according to claim 1 or claim 2.

4. The bacterial strain of claim 3 comprising *Agrobacterium tumefaciens* or *Agrobacterium rhizogenes.*

5. A method for genetically modifying a plant cell, comprising the steps of:
(a) inserting a plant structural gene into T-DNA comprising the promoter of the "1.6" transcript of T-DNA, the promoter and the plant structural gene being in such a position and orientation with respect to each other that the plant structural gene is expressible in a plant cell under the control of the T-DNA promoter, and the plant structural gene comprising an intron, thereby forming a T-DNA promoter/plant structural gene combination, and then
(b) transferring the T-DNA promoter/plant structural gene combination into a plant cell.

6. A method according to claim 5, further comprising after execution of step (b) the step of:
(c) detecting expression of the plant structural gene in a plant cell containing the T-DNA promoter/plant structural gene combination.

7. A method according to claim 5, wherein the plant structural gene encodes a seed storage protein.

8. A method according to claim 5, wherein the plant structural gene encodes phaseolin.

9. A method according to claim 5, wherein the T-DNA promoter/plant structural gene combination is maintained and replicated prior to step (b) as part of a shuttle vector.

10. A method according to claim 5, wherein the plant structural gene is fused to a T-DNA structural gene, whereby the T-DNA structural gene/plant structural gene combination encodes a fusion protein comprising T-DNA encoded protein and plant protein sequences.

**11.** A method according to claim 5, wherein the cell is from a dicotyledonous plant.

**12.** A method according to claim 11, wherein the dicotyledonous plant is a member of the *Compositeae* or the *Leguminoseae.*

**13.** A plant tissue or a plant cell produced according to the method of any of claims 5-12.

**14.** A plant grown from a plant tissue or plant cell according to claim 13.

**Patentansprüche**

**1.** DNA-Vektor, umfassend eine T-DNA mit einem darin eingefügten unter der Kontrolle des Promotors des "1,6"-Transkripts der T-DNA stehenden Pflanzenstrukturgen, wobei der Promotor und das Pflanzenstrukturgen in solcher Lage und Orientierung zu einander sind, daß das Pflanzenstrukturgen in einer Pflanzenzelle unter der Kontrolle des T-DNA-Promotors exprimierbar ist, und das Pflanzenstrukturgen ein Intron umfaßt.

**2.** DNA-Vektor nach Anspruch 1, worin die T-DNA eine inaktivierende Mutation in tms oder tmr enthält.

**3.** Bakterienstamm, der einen Vektor nach Anspruch 1 oder 2 enthält und repliziert.

**4.** Bakterienstamm nach Anspruch 3, umfassend Agrobacterium tumefaciens oder Agrobacterium rhizogenes.

**5.** Verfahren zur genetischen Modifizierung einer Pflanzenzelle, bei dem
(a) ein Pflanzenstrukturgen in eine den Promotor des "1,6" Transkripts der T-DNA umfassende T-DNA eingefügt wird, wobei der Promotor und das Pflanzenstrukturgen in solcher Lage und Orientierung zueinander sind, daß das Pflanzenstrukturgen in einer Pflanzenzelle unter der Kontrolle des T-DNA-Promotors exprimierbar ist, und das Pflanzenstrukturgen ein Intron umfaßt, wodurch eine T-DNA-Promotor/Pflanzenstrukturgen-Kombination gebildet wird und dann
(b) die T-DNA-Promotor/Pflanzenstrukturgen-Kombination in eine Pflanzenzelle transferiert wird.

**6.** Verfahren nach Anspruch 5, bei dem ferner nach Durchführung von Schritt (b)
(c) die Expression des Pflanzenstrukturgens in einer die T-DNA-Promotor/Pflanzenstrukturgen-Kombination enthaltenden Pflanzenzelle nachgewiesen wird.

**7.** Verfahren nach Anspruch 5, worin das Pflanzenstrukturgen ein Saatlagerungs-Protein codiert.

**8.** Verfahren nach Anspruch 5, worin das Pflanzenstrukturgen Phaseolin codiert.

**9.** Verfahren nach Anspruch 5, worin die T-DNA-Promotor/ Pflanzenstrukturgen-Kombination stabil gehalten und vor Schritt (b) als Teil eines Shuttle-Vektors repliziert wird.

**10.** Verfahren nach Anspruch 5, worin das Pflanzenstrukturgen mit einem T-DNA-Strukturgen fusioniert wird, wodurch die T-DNA-Strukturgen/Pflanzenstrukturgen-Kombination ein Fusionsprotein codiert, das T-DNA codierte Protein- und Pfanzenproteinsequenzen umfaßt.

**11.** Verfahren nach Anspruch 5, worin die Zelle von einer dikotyledonen Pflanze stammt.

**12.** Verfahren nach Anspruch 11, worin die dikotyledone Pflanze ein Mitglied der Familie Compositeae oder Leguminoseae ist.

**13.** Pflanzengewebe oder Pflanzenzelle, hergestellt nach dem Verfahren nach einem der Ansprüche 5 - 12.

**14.** Pflanze, gewachsen von einem Pflanzengewebe oder einer Pflanzenzelle nach Anspruch 13.

## Revendications

1. Vecteur d'ADN comprenant un T-ADN dans lequel est introduit un gène structurel végétal sous le contrôle du promoteur du transcript " 1.6" du T-ADN, le promoteur et le gène structurel végétal étant dans une position et une orientation telles l'un vis-à-vis de l'autre que le gène structurel végétal puisse être exprimé dans une cellule végétale sous le contrôle du promoteur du T-ADN, et le gène structurel végétal comprenant un intron.

2. Vecteur d'ADN selon la revendication 1, dans lequel le T-ADN contient une mutation inactivante dans tms ou tmr.

3. Souche bactérienne contenant et répliquant un vecteur selon la revendication 1 ou 2.

4. Souche bactérienne selon la revendication 3 comprenant Agrobacterium tumefaciens ou Agrobacterium rhizogenes.

5. Procédé de modification génétique d'une cellule végétale , comprenant les étapes suivantes :
   (a) on insère un gène structurel végétal dans du T-ADN comprenant le promoteur du transcript "1.6" du T-ADN, le promoteur et le gène structurel végétal étant dans une position et une orientation telles l'un par rapport à l'autre que le gène structurel végétal puisse être exprimé dans une cellule végétale sous le contrôle du promoteur du T-ADN, et le gène structurel végétal comprenant un intron,afin de former une combinaison du promoteur du T-ADN et du gène structurel végétal, et ensuite
   (b) on transfère la combinaison de promoteur du T-ADN et du gène structurel végétal dans une cellule végétale .

6. Procédé selon la revendication 5 , comprenant, après exécution de l'étape (b), l'étape suivante :
   (c) on détecte l'expression du gène structurel végétal dans une cellule végétale contenant la combinaison du promoteur de T-ADN et du gène structurel végétal.

7. Procédé selon la revendication 5, dans lequel le gène structurel végétal code une protéine de stockage de semence.

8. Procédé selon la revendication 5, dans lequel le gène structurel végétal code la phaséoline.

9. Procédé selon la revendication 5, dans lequel la combinaison du promoteur de T-ADN et du gène structurel végétal est maintenue et répliquée avant l'étape (b) en tant que partie d'un vecteur navette .

10. Procédé selon la revendication 5, dans lequel le gène structurel végétal est fusionné avec un gène structurel du T-ADN, en sorte que la combinaison du gène structurel du T-ADN et du gène structurel végétal code une protéine de fusion comprenant des séquences de protéines codées par le T-ADN et de protéines végétales.

11. Procédé selon la revendication 5, dans lequel la cellule est issue d'une plante de type dicotylédone.

12. Procédé selon la revendication 11, dans lequel la plante de type dicotylédone est un membre des Compositeae ou des Laguminoseae.

13. Tissu végétal ou cellule végétale produit(e) selon le procédé de l'une quelconque des revendications 5 à 12.

14. Plante développée à partir d'un tissu végétal ou d'une cellule végétale selon la revendication 13.

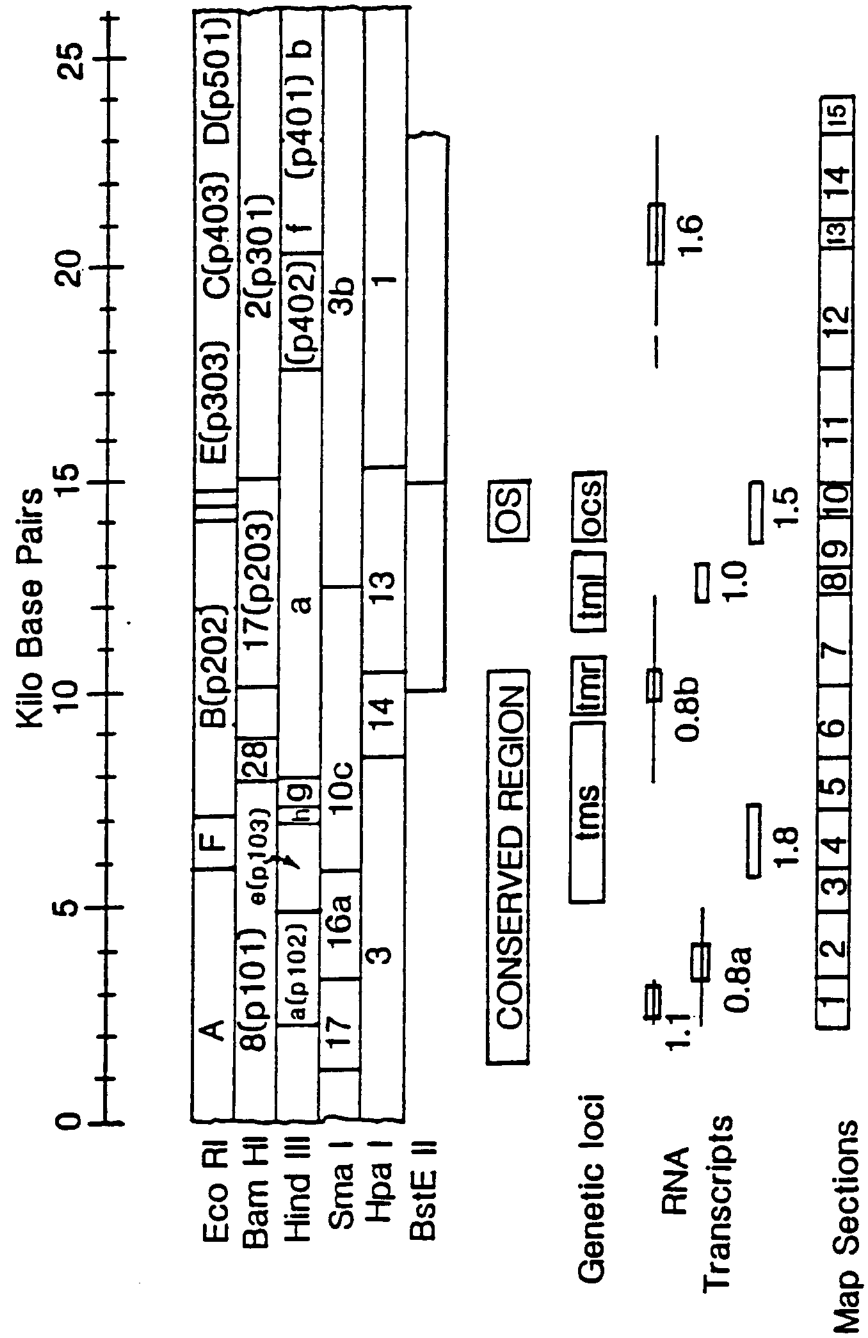


T-DNA REGION OF pTi15955

FIG. I

# FIG. 2 COMPLETE SEQUENCE OF NOPALINE SYNTHASE

```
TGATAGTTTAAACCGAAGGCGGGAAACGACAATCTGATCATGAGCGGAGAATTAAGGGAGTCACGTTATGACCCCCGCCGATGACGCGGGACAAGCCGTT 100

TTACGTTTGGAACTGACAGAACCGCAACGTTGAAGGAGCCACTGAGCCGCGGGTTTCTGGAGTTTAATGAGCTAAGCACATACGTCAGAAACCATTATTG 200

CGCGTTCAAAAGTCGCCTAAGGTCACTATCAGCTAGCAAATATTTCTTGTCAAAAATGCTCCACTGACGTTCCATAAATTCCCCTCGGTATCCAATTAGA 300

GTCTCATATTCACTCTCAATCCAAATAATCTGCAATGGCAATTACCTTATCCGCAACTTCTTTACCTATTTCCGCCCGCAGATCACCATCCGCTTCCCTTG 400
                                                  MetAlaIleThrLeuSerAlaThrSerLeuProIleSerAlaAlaAspHisHisProLeuProLeu

ACCGTAGGTGTCCTCGGTTCTGGTCACGCGGGGACTGCATTAGCGGCTTGGTTCGCCTCCCGGCACGTTCCCACGGCGCTGTGGGCACCAGCAGATCATC 500
ThrValGlyValLeuGlySerGlyHisAlaGlyThrAlaLeuAlaAlaTrpPheAlaSerArgHisValProThrAlaLeuTrpAlaProAlaAspHisP

CAGGATCGATCTCAGCAATCAAGGCCAGTGAAGGAGTTATCACCACCGAGGGAATGATTAACGGTCCATTTAGGGTCTCAGCCTGTGATGACCTTGCCGC 600
roGlySerIleSerAlaIleLysAlaSerGluGlyValIleThrThrGluGlyMetIleAsnGlyProPheArgValSerAlaCysAspAspLeuAlaAl

AGTTATTCGCTCCAGCCGTGTACTGATTATTGTAACCCGTGCGGACGTTCACGACAGCTTCGTCAACGAACTCGCCAACTTCAACGGCGAACTCGCAACA 700
aValIleArgSerSerArgValLeuIleIleValThrArgAlaAspValHisAspSerPheValAsnGluLeuAlaAsnPheAsnGlyGluLeuAlaThr

AAGGATATTGTCGTCGTGTGCGGCCATGGCTTCTCCATCAAGTACGAGAGACAGCTGCGATTCAAGCGAATATTCGAGACGGATAATTCGCCCATAACGT 800
LysAspIleValValValCysGlyHisGlyPheSerIleLysTyrGluArgGlnLeuArgPheLysArgIlePheGluThrAspAsnSerProIleThrS

CTAAGCTATCGGATCAAAAAAAATGTAACGTCAACATCAAGGAAATGAAAGCGTCTTTCGGACTGTCATGTTTCCCAATTCATCGCGATGATGCTGGCGT 900
erLysLeuSerAspGlnLysLysCysAsnValAsnIleLysGluMetLysAlaSerPheGlyLeuSerCysPheProIleHisArgAspAspAlaGlyVa

GATTGATCTACCCGAAGATACCAAGAACATCTTTGCCCAGCTATTTTCCGCTAGAATCATCTGCATCCCGCCGTTGCAAGTGCTATTCTTTTCCAACTAT 1000
lIleAspLeuProGluAspThrLysAsnIlePheAlaGlnLeuPheSerAlaArgIleIleCysIleProProLeuGlnValLeuPhePheSerAsnTyr

ATCACTCATGCGGTTCCGGCAGTCATGAACATCGGAAGACTCCGCGACCCAGCCAATTCTCTTACTAAAAGAGCTGAGAAGTGGCTTCTTGAACTAGACG 1100
IleThrHisAlaValProAlaValMetAsnIleGlyArgLeuArgAspProAlaAsnSerLeuThrLysArgAlaGluLysTrpLeuLeuGluLeuAspG

AGCGAACCCCCACGAGCCGAGAAGGGCTTTTTCTTTTATGGTGAAGGATCCAACACTTACGTTTGCAACGTCCAAGAGCAAATAGACCACGAACGCCGGAA 1200
luArgThrProArgAlaGluLysGlyPhePhePheTyrGlyGluGlySerAsnThrTyrValCysAsnValGlnGluGlnIleAspHisGluArgArgLy

GGTTGCCGCAGCGTGTGGACTGCGTCTCAATTCTCTCTTGCAGGAATGCAATGATGAATATGATACTGACTATGAAACTTTGAGGGAATACTGCCTAGCA 1300
sValAlaAlaAlaCysGlyLeuArgLeuAsnSerLeuLeuGlnGluCysAsnAspGluTyrAspThrAspTyrGluThrLeuArgGluTyrCysLeuAla

CCGTCACCTCATAACGTGCATCATGCATGCCCTGACAACATGGAACATCGCTATTTTTCTGAAGAATTATGCTCGTTGGAGGATGTCGCGGCAATTGCAG 1400
ProSerProHisAsnValHisHisAlaCysProAspAsnMetGluHisArgTyrPheSerGluGluLeuCysSerLeuGluAspValAlaAlaIleAlaA

CTATTGCCAAAATCGAAATACCCCTCACGCATGCATTCATCAATATTATTCATGCGGGGAAAGGCAAGATTAATCCAACTGGCAAATCATCCAGCGTGAT 1500
laIleAlaLysIleGluIleProLeuThrHisAlaPheIleAsnIleIleHisAlaGlyLysGlyLysIleAsnProThrGlyLysSerSerSerValIl

TGGTAACTTCAGTTCCAGCGACTTGATTCGTTTTGGTGCTACCCACGTTTTCAATAAGGACGAGATGGTGGAGTAAAGAAGGAGTGCGTCGAAGCAGATC 1600
eGlyAsnPheSerSerSerAspLeuIleArgPheGlyAlaThrHisValPheAsnLysAspGluMetValGluEnd

GTTCAAACATTTGGCAATAAAGTTTCTTAAGATTGAATCCTGTTGCCGGTCTTGCGATGATTATCATATAATTTCTGTTGAATTACGTTAAGCATGTAAT 1700

AATTAACATGTAATGCATGACGTTATTTATGAGATGGGTTTTTATGATTAGAGTCCCGCAATTATACATTTAATACGCGATAGAAAACAAAATATAGCGC 1800
```

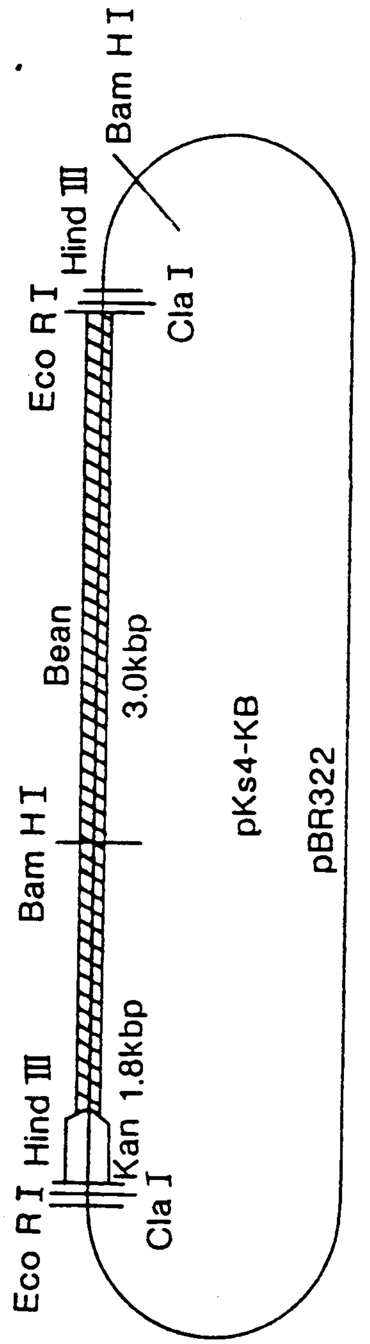
Bam H I
Hind III
Eco R I
Cla I
Bean
3.0kbp
Bam H I
pKs4-KB
pBR322
Hind III
Kan 1.8kbp
Eco R I
Cla I

FIG. 3

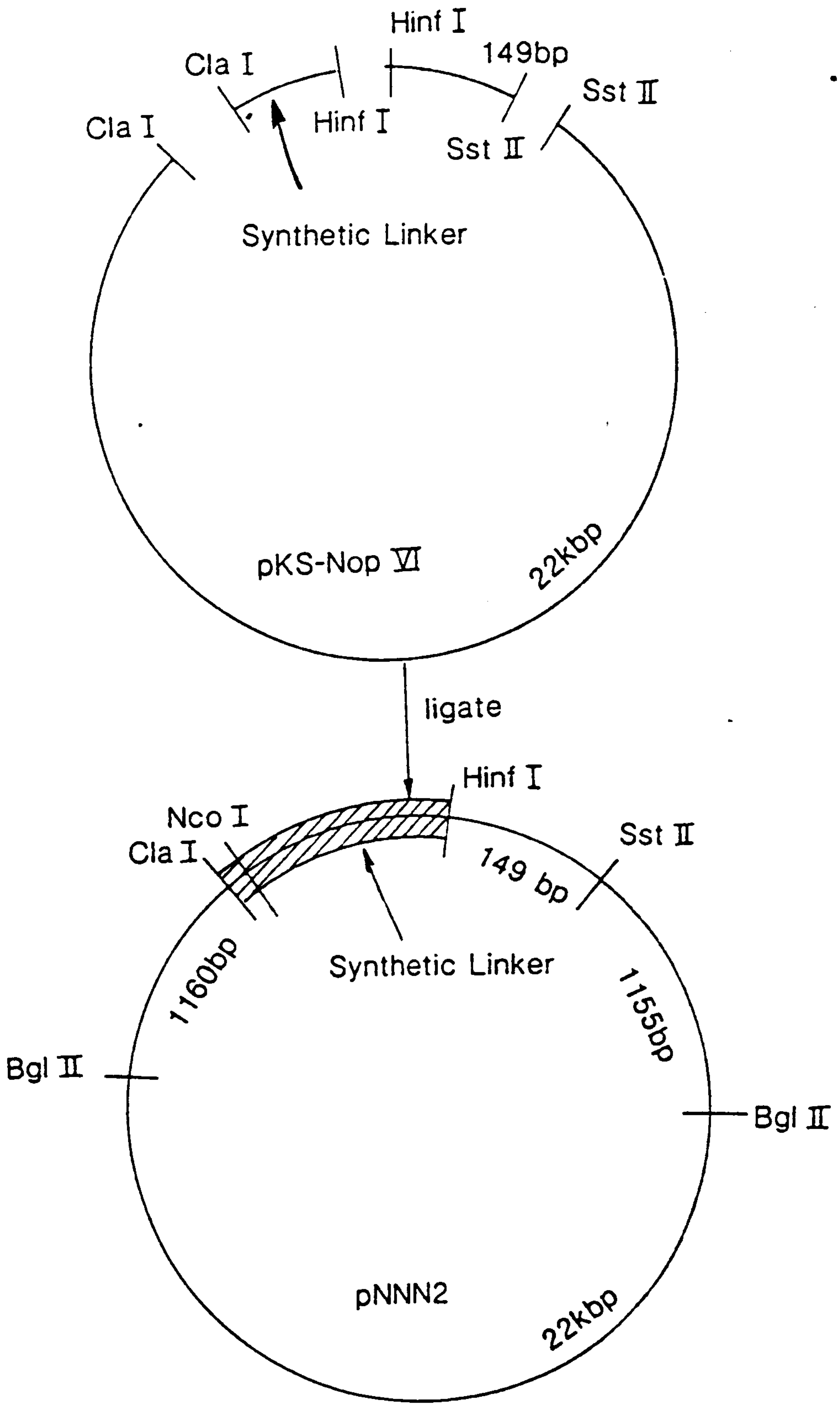
Hinf I
Cla I
149bp
Sst II
Cla I
Hinf I
Sst II
Synthetic Linker
pKS-Nop VI
22kbp
ligate
Hinf I
Nco I
Sst II
Cla I
149 bp
1160bp
Synthetic Linker
1155bp
Bgl II
Bgl II
pNNN2
22kbp

FIG. 4

FIG. 5-1

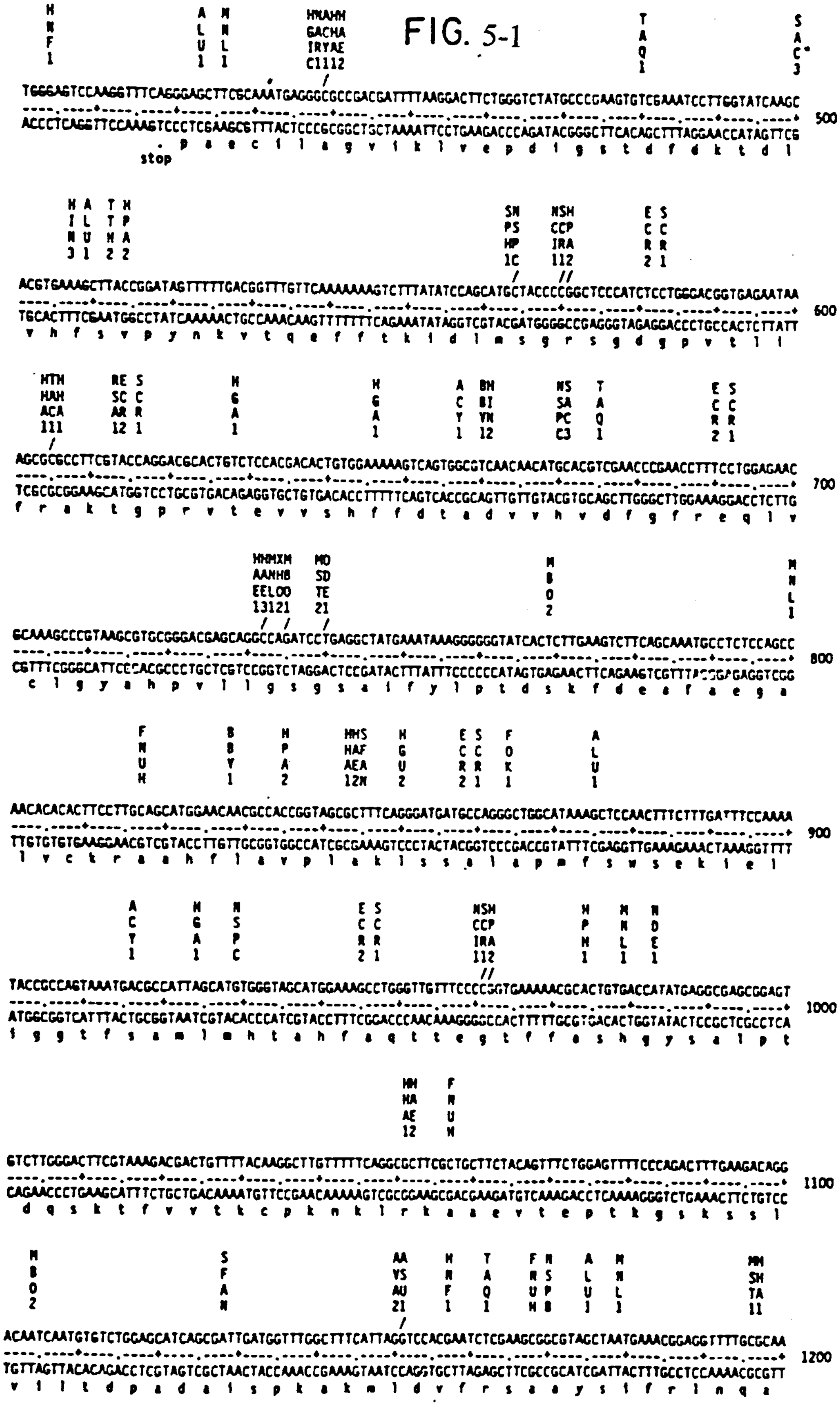

FIG. 5-2

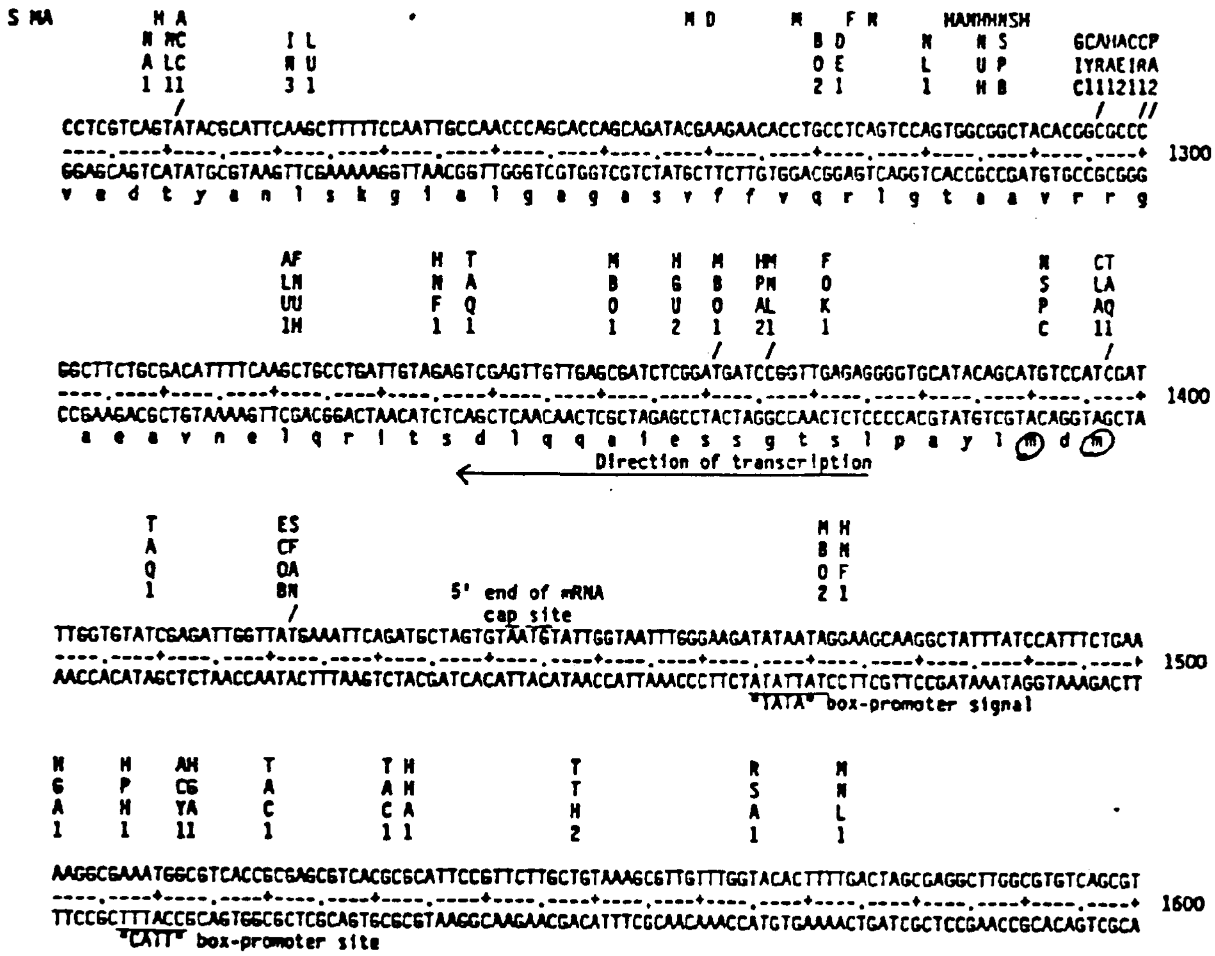
1300
1400
Direction of transcription
5' end of mRNA
cap site
1500
"TATA" box-promoter signal
1600
"CATT" box-promoter site

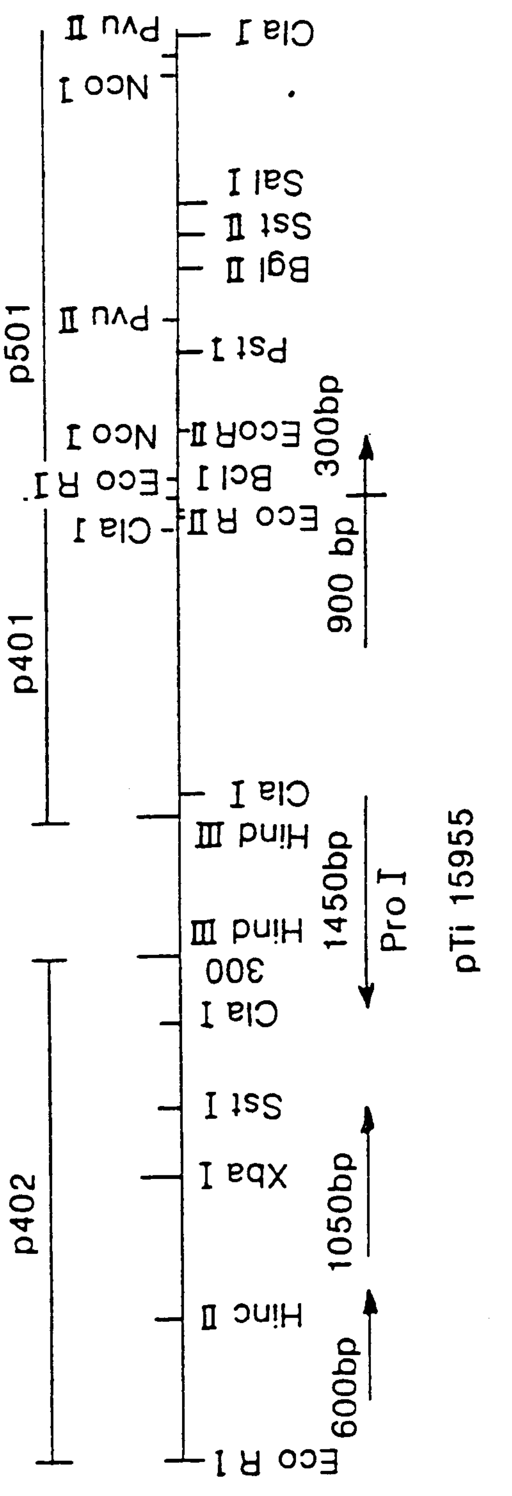
p402
p401
p501
Eco R I
Hinc II
Xba I
Sst I
Cla I
300
Hind III
Hind III
Cla I
Eco R II
Bcl I
EcoR II
Pst I
Bgl II
Sst II
Sal I
Cla I
Pvu II
Nco I
Eco R I
Cla I
Nco I
Pvu II
600bp
1050bp
1450bp
Pro I
900 bp
300bp
pTi 15955

FIG. 6

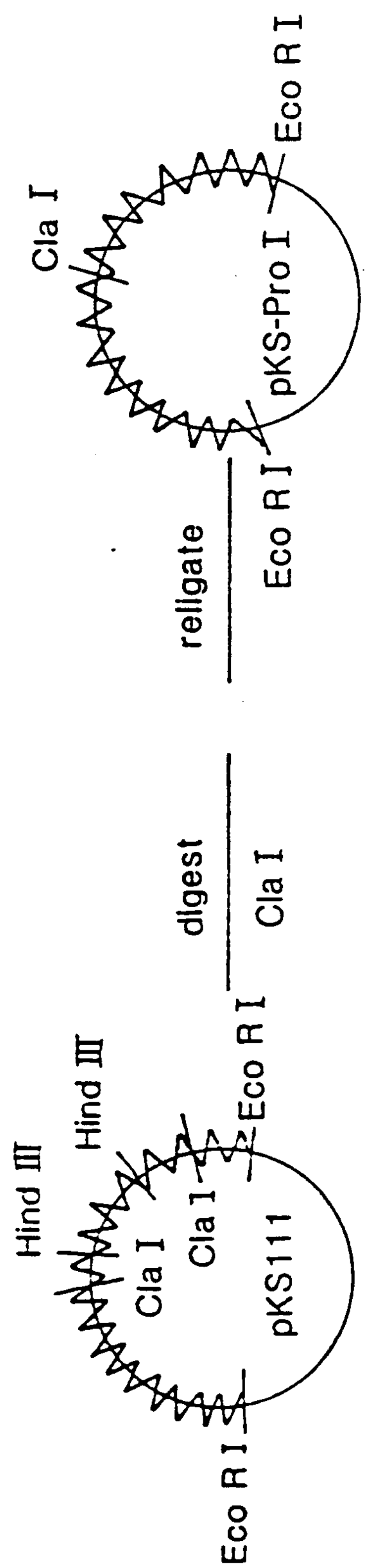
Eco R I
pKS111
Cla I
Cla I
Hind III
Hind III
Eco R I
digest
Cla I
religate
Eco R I
pKS-Pro I
Cla I
Eco R I

FIG. 7

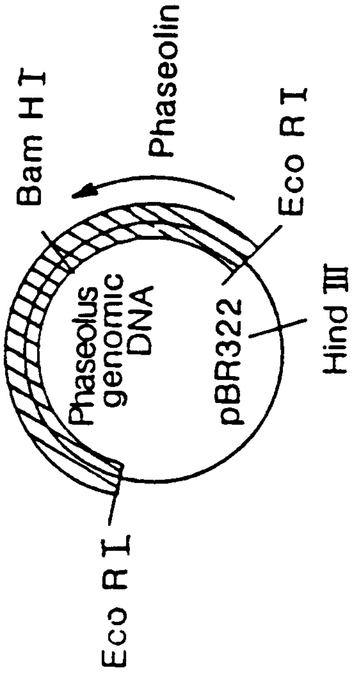


p 7.2

FIG 8

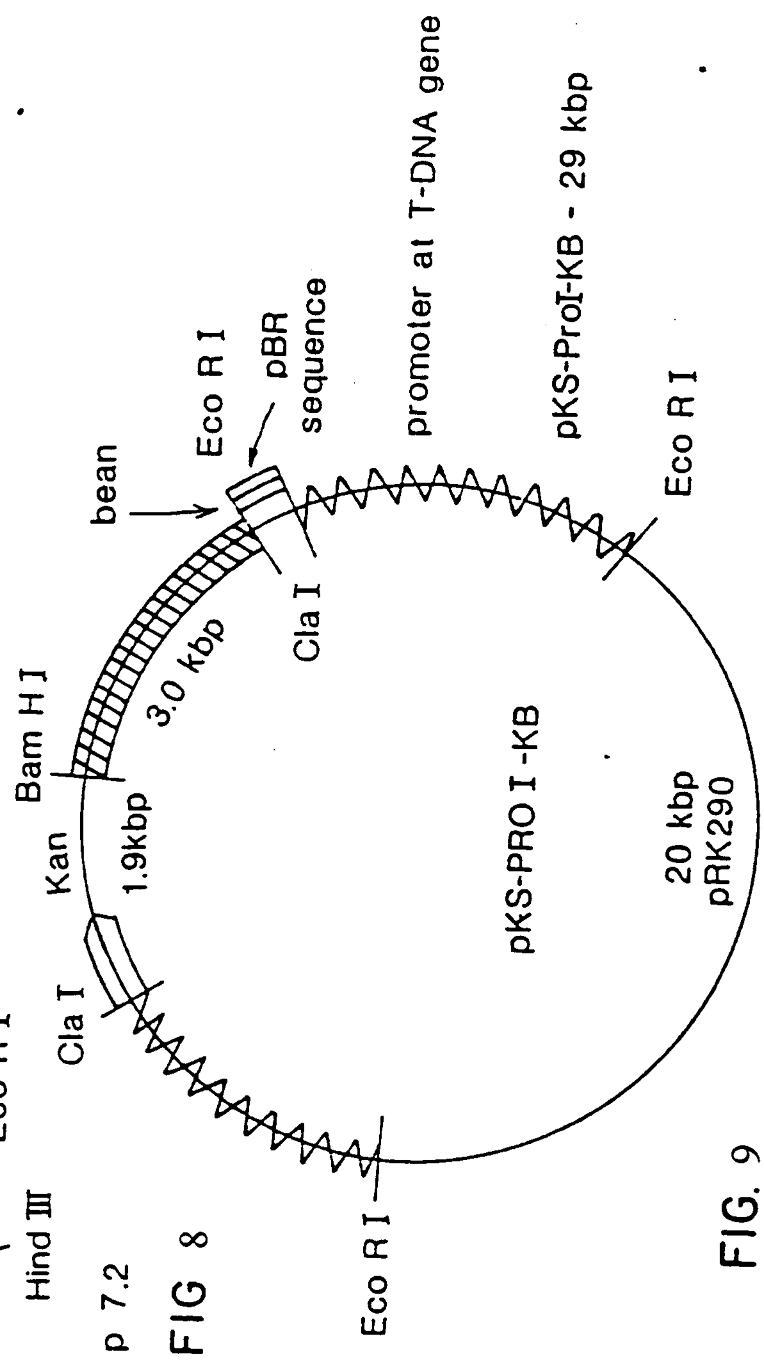


FIG. 9

STRUCTURE of the PHASEOLIN STORAGE PROTEIN GENE

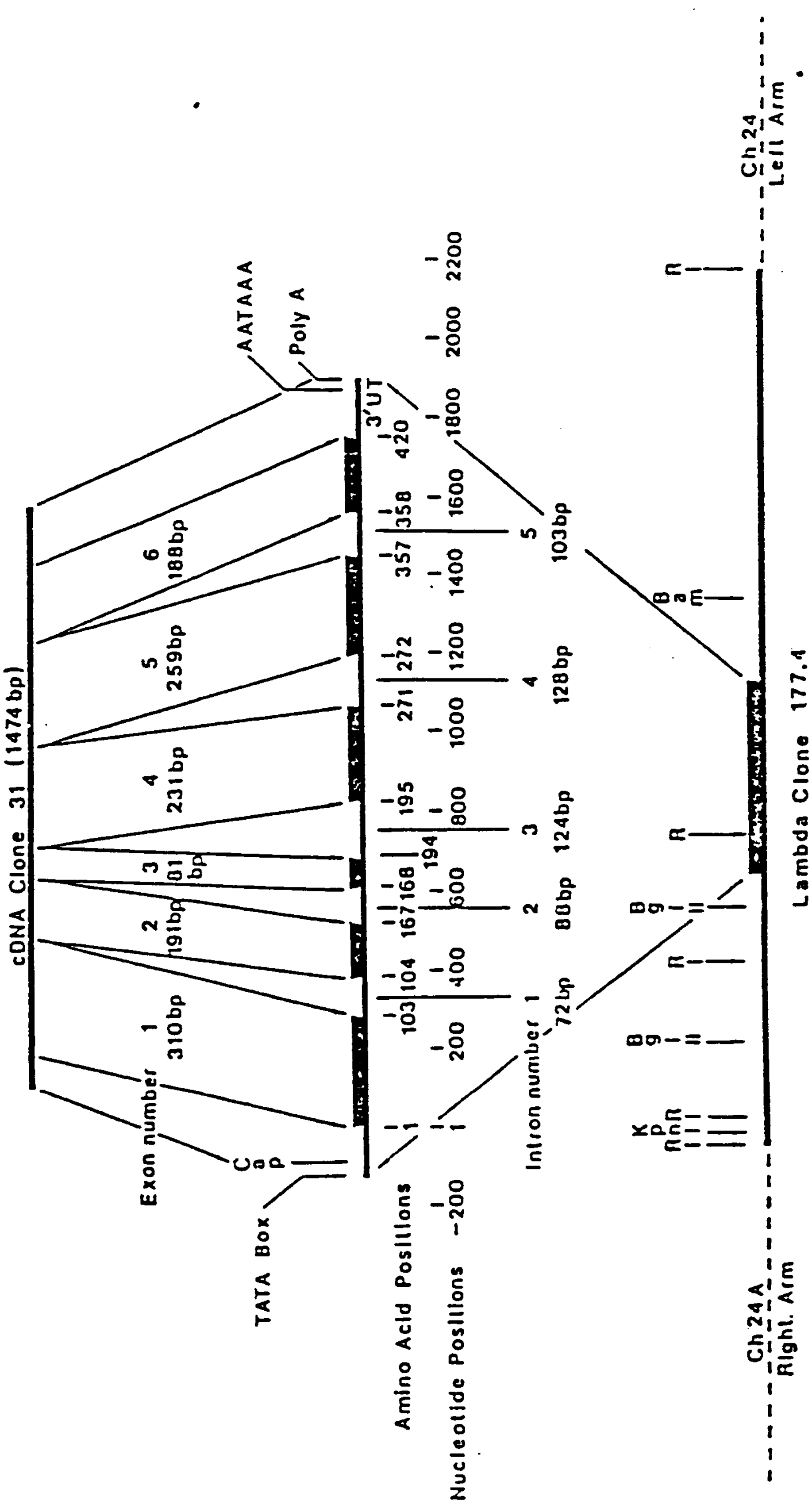


FIG. 10

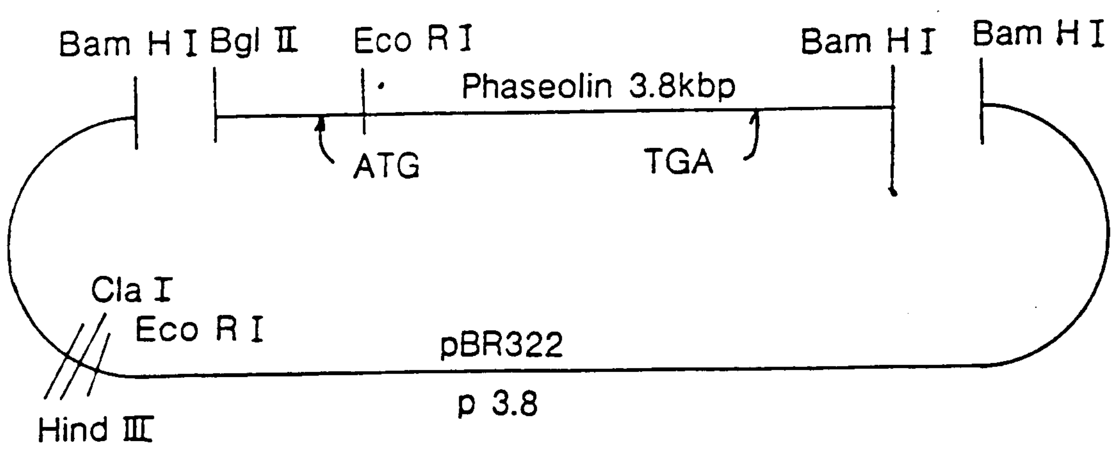


FIG. 11

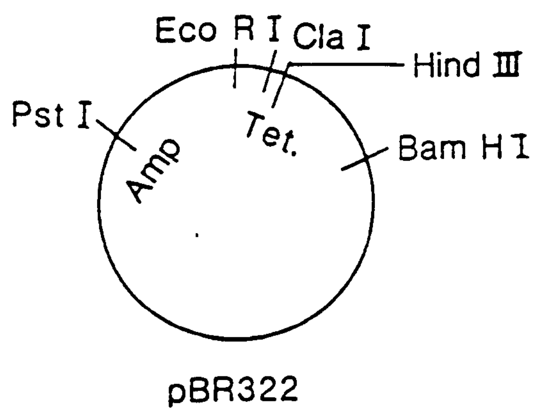


FIG. 12

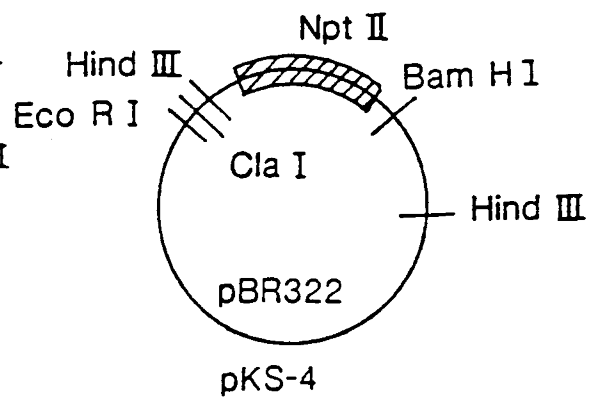


FIG. 13

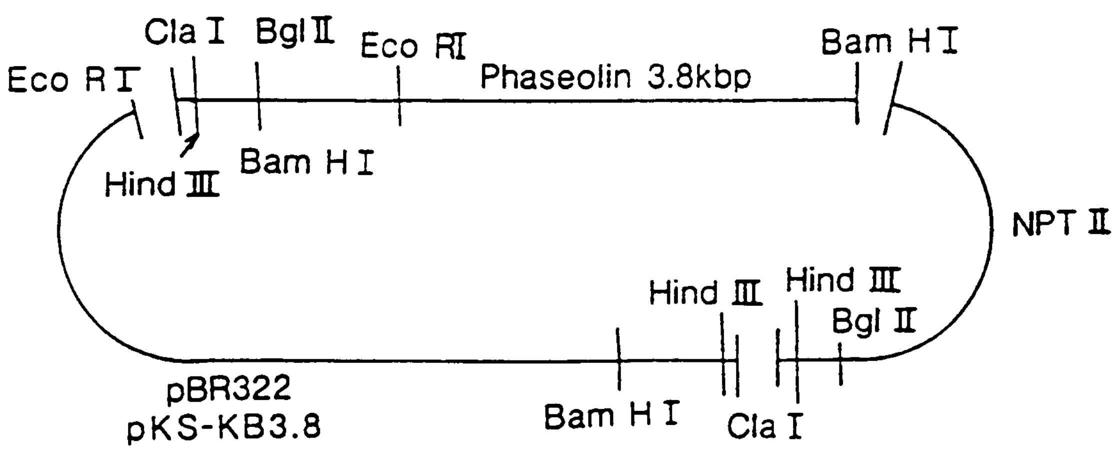


FIG. 14

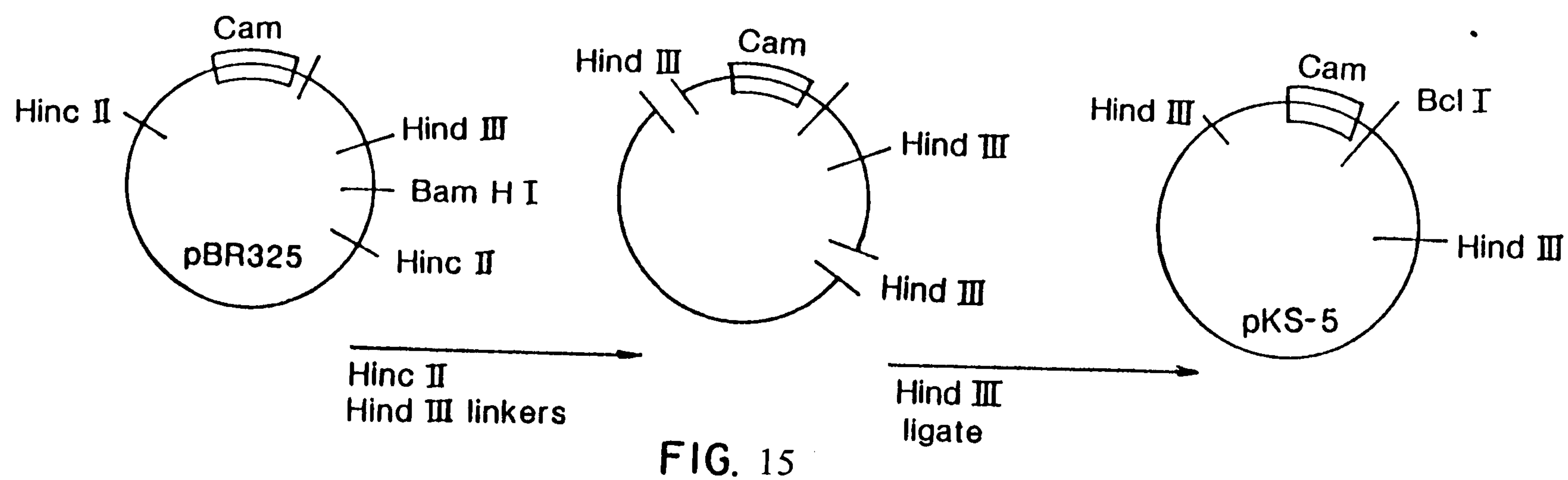
Cam
Hinc II
Hind III
Bam H I
Hinc II
pBR325
Hinc II
Hind III linkers
Hind III
Cam
Hind III
Hind III
Hind III
ligate
Cam
Hind III
Bcl I
Hind III
pKS-5

FIG. 15

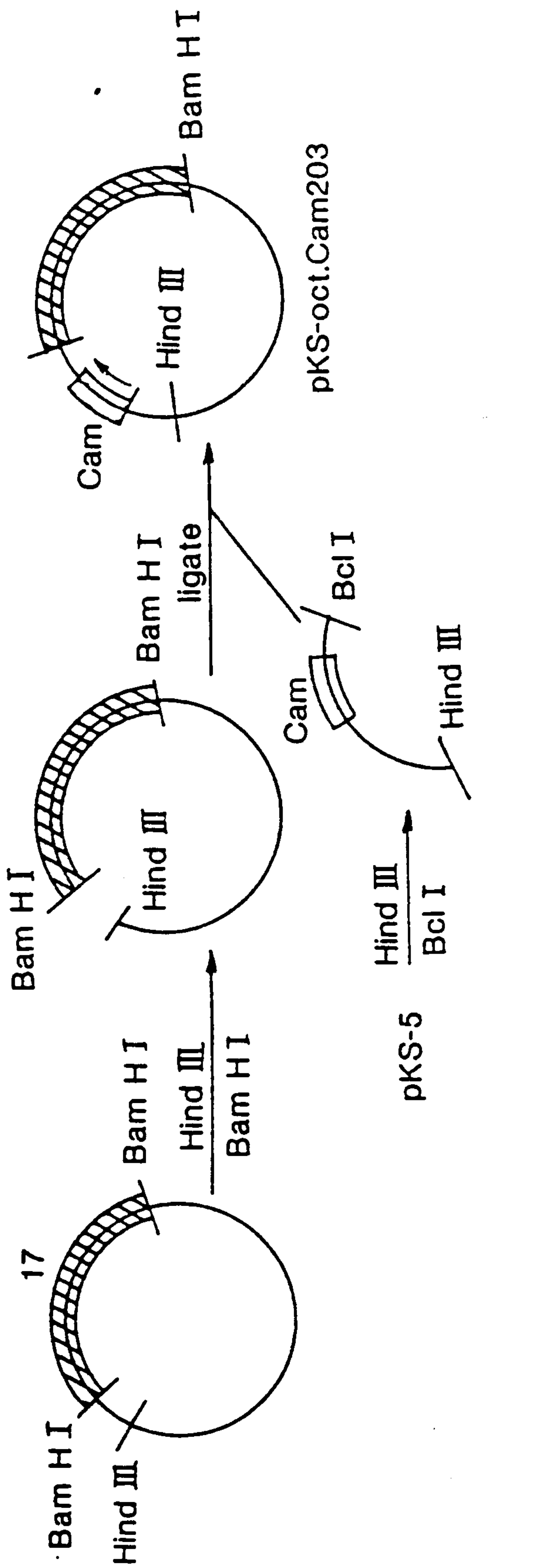
17
Bam H I
Hind III
Bam H I
Hind III
Bam H I
Bam H I
Hind III
Bam H I
Hind III
Bam H I
pKS-5
Hind III
Bcl I
Cam
Bcl I
Hind III
Bam H I
ligate
Cam
Hind III
Bam H I
pKS-oct.Cam203

FIG. 16

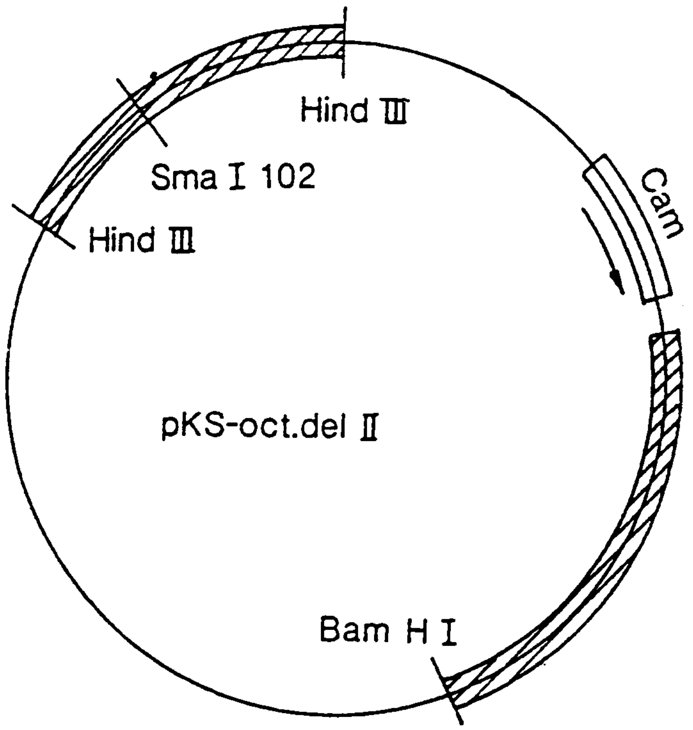
Hind III
Sma I 102
Hind III
Cam
pKS-oct.del II
Bam H I

FIG. 17

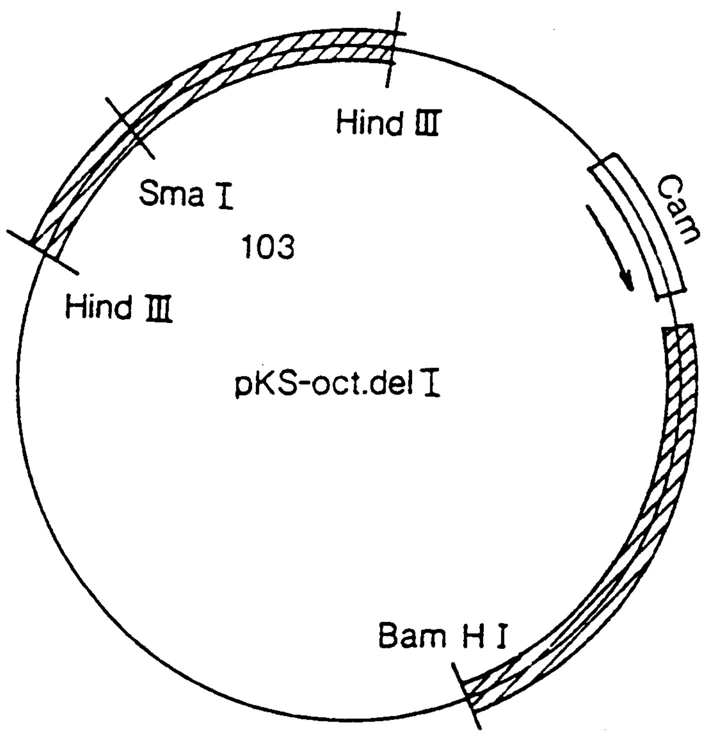
Hind III
Sma I
103
Hind III
Cam
pKS-oct.del I
Bam H I

FIG. 18

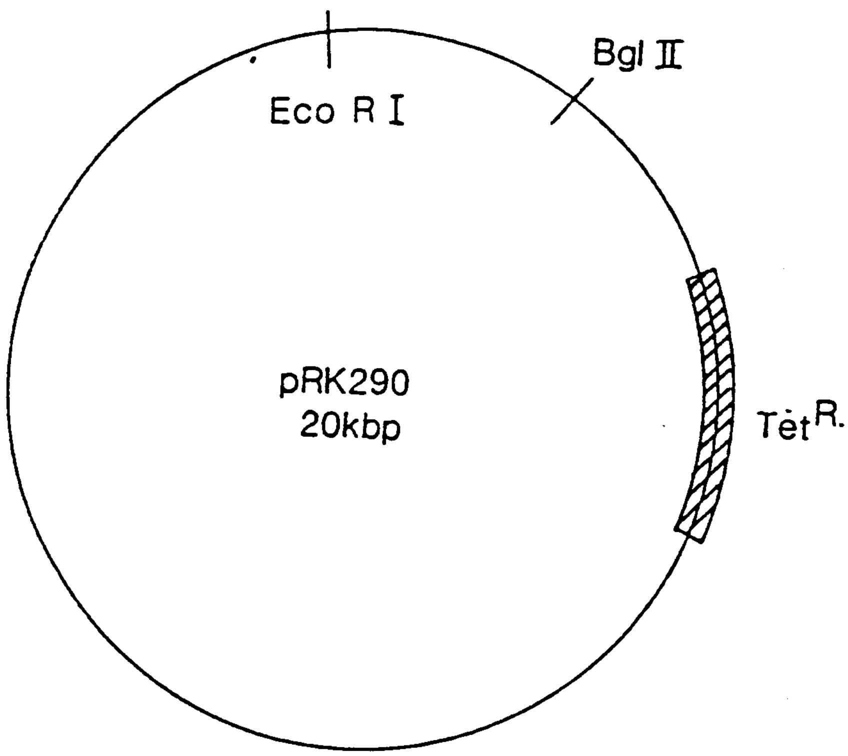


FIG. 19

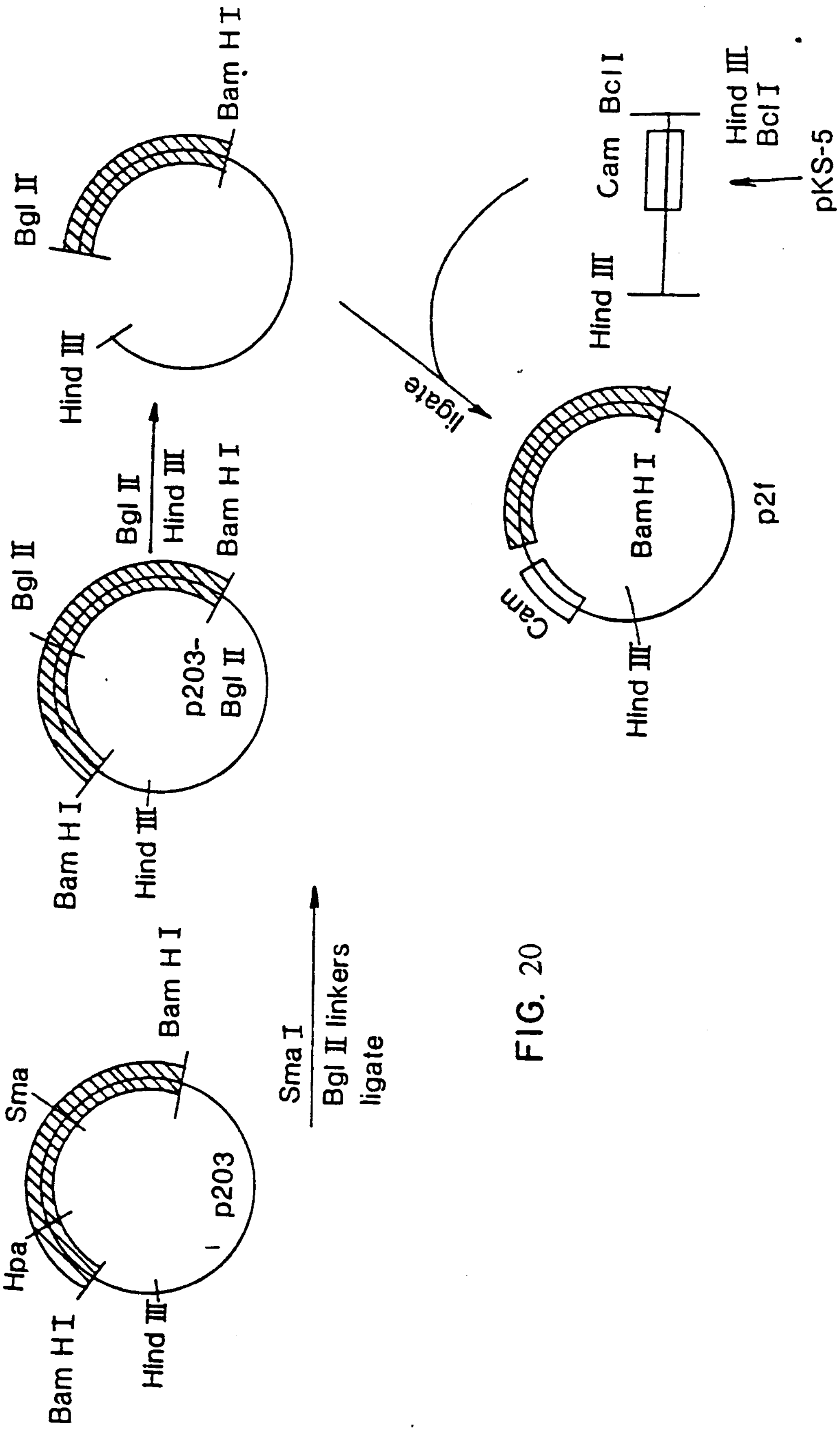
Bam H I
Hpa
Sma
Hind III
Bam H I
p203
Sma I
Bgl II linkers
ligate
Bam H I
Hind III
Bgl II
Bam H I
p203-
Bgl II
Bgl II
Hind III
Bam H I
Hind III
Bgl II
Bam H I
ligate
Hind III
Cam
Bcl I
Hind III
Bcl I
pKS-5
Cam
Hind III
Bam H I
p21

FIG. 20

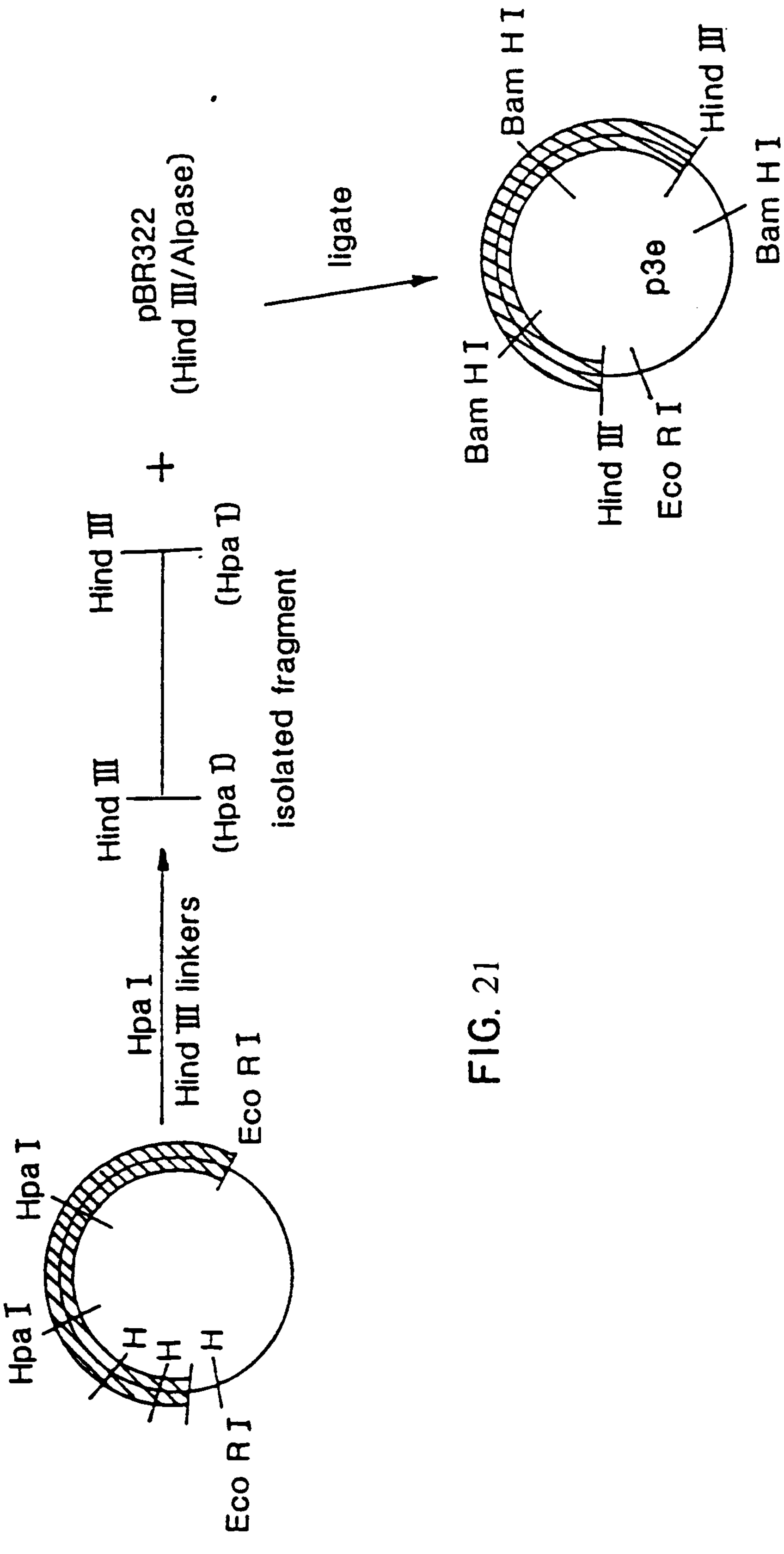
Hpa I
Hpa I
H
H
H
Eco R I
Eco R I
Hpa I
Hind III linkers
Hind III
Hind III
(Hpa I)
(Hpa I)
isolated fragment
+
pBR322
(Hind III/Alpase)
ligate
Bam H I
Hind III
Bam H I
p3e
Bam H I
Hind III
Eco R I

FIG. 21

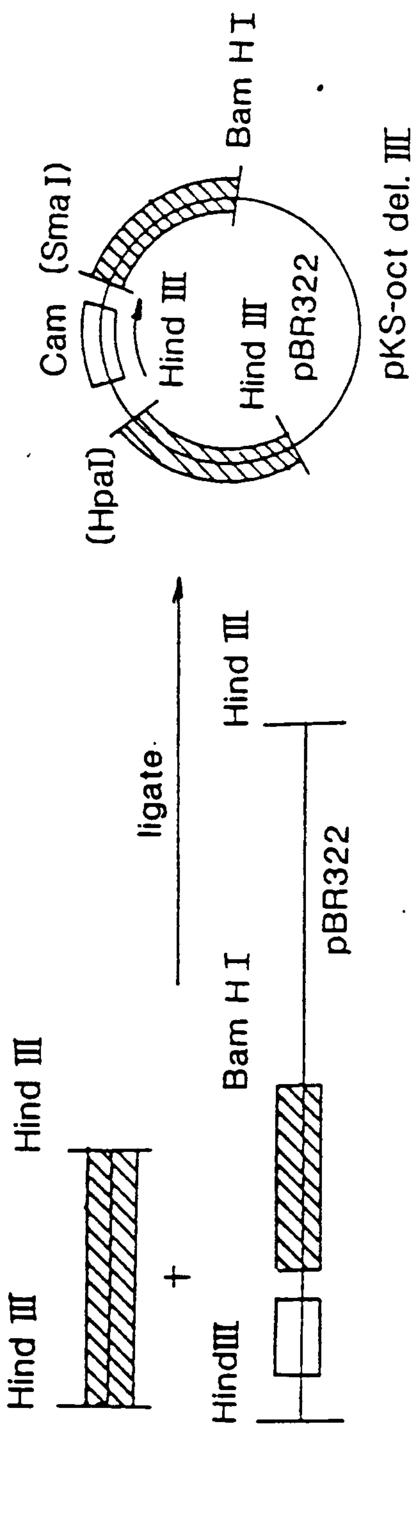
Hind III
Hind III
+
HindIII
Bam H I
pBR322
Hind III
ligate
(HpaI)
Cam
(Sma I)
Hind III
Hind III
pBR322
Bam H I
pKS-oct del. III

FIG. 22

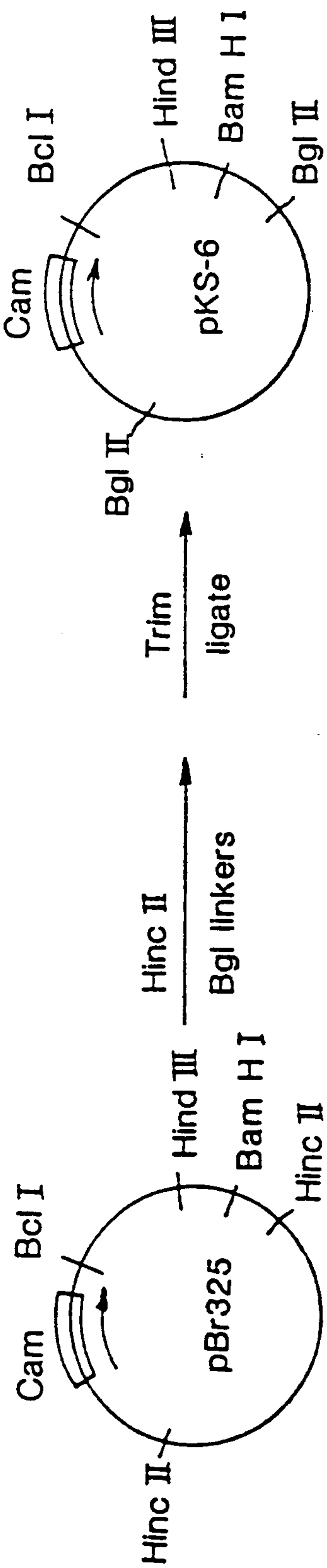
Hinc II
Cam
Bcl I
pBr325
Hind III
Bam H I
Hinc II
Hinc II
Bgl linkers
Trim
ligate
Bgl II
Cam
Bcl I
pKS-6
Hind III
Bam H I
Bgl II

FIG. 23

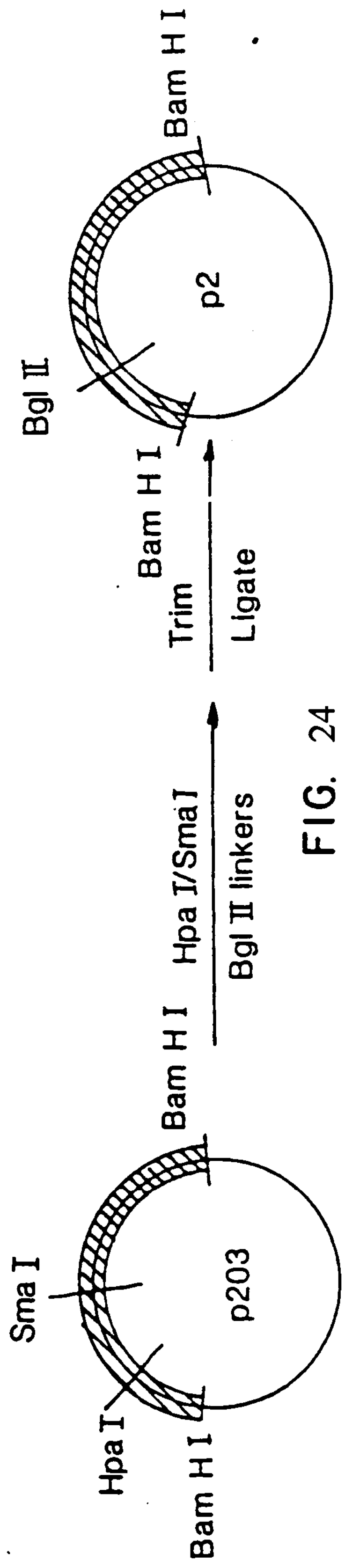

p203
Sma I
Hpa I
Bam H I
Bam H I
Hpa I/Sma I
Bgl II linkers
Trim
Ligate
Bam H I
Bgl II
Bam H I
p2


FIG. 24

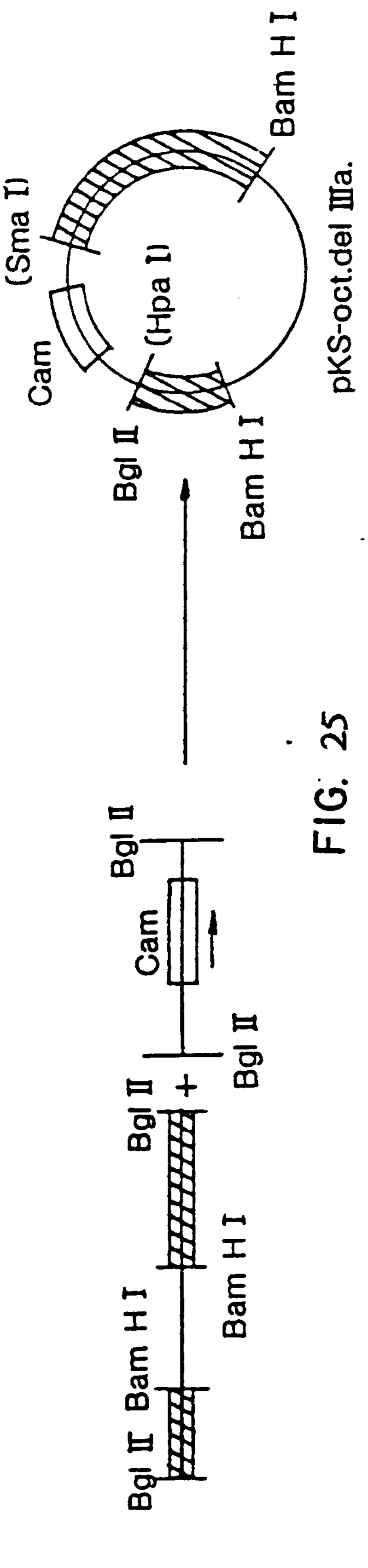

Bgl II
Bam H I
Bam H I
Bam H I
Bgl II
+
Bgl II
Bgl II
Cam
Bgl II
Bgl II
Bam H I
Cam
(Sma I)
(Hpa I)
Bam H I
pKS-oct.del IIIa.


FIG. 25

FIG. 26

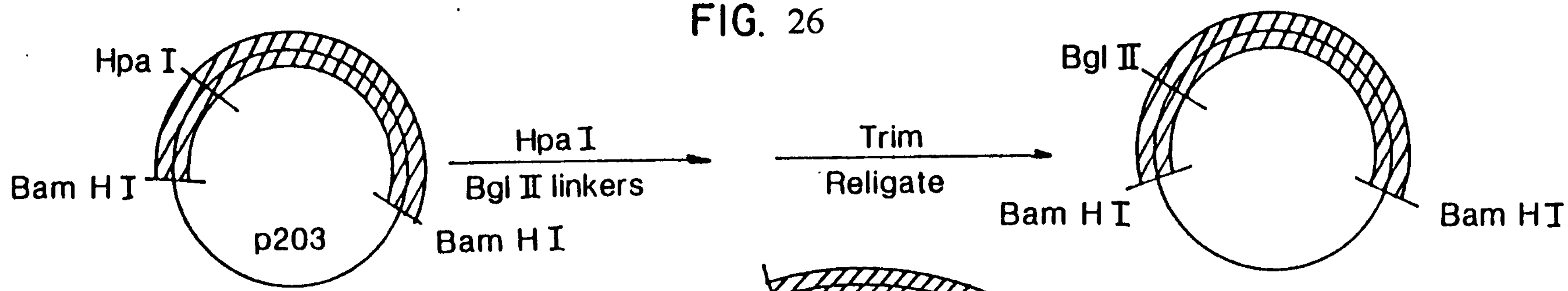


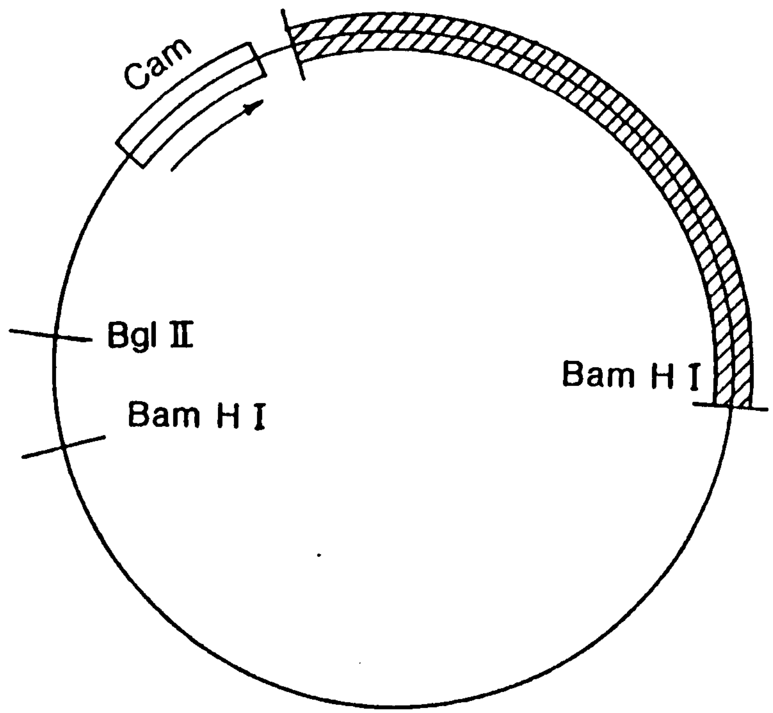


pKS-oct.tmr.

FIG. 27

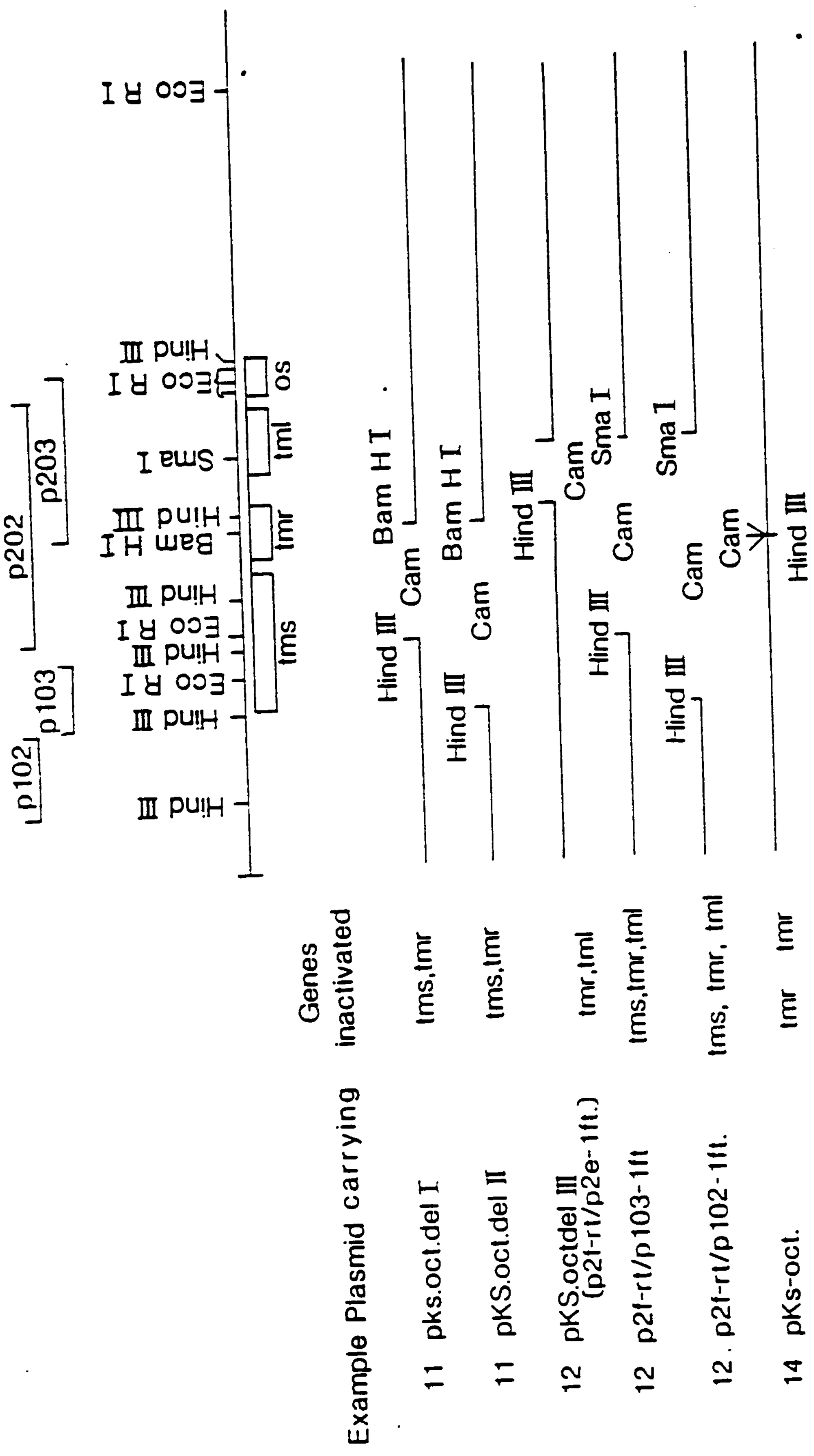

p102
p103
p202
p203
Hind III
Eco R I
Hind III
Eco R I
Hind III
Bam H I
Hind III
Sma I
Eco R I
Hind III
Eco R I
tms
tmr
tml
os
Hind III
Cam
Bam H I
Cam
Hind III
Cam
Sma I
Cam
Sma I
Cam
Hind III
Example Plasmid carrying
Genes inactivated
11 pks.oct.del I
tms,tmr
11 pKS.oct.del II
tms,tmr
12 pKS.octdel III (p2f-rt/p2e-1ft.)
tmr,tml
12 p2f-rt/p103-1ft
tms,tmr,tml
12. p2f-rt/p102-1ft.
tms, tmr, tml
14 pKs-oct.
tmr tmr


FIG. 28

GENETIC CODE

| 1st BASE / 2nd BASE | U | C | A | G | 3rd BASE |
|---|---|---|---|---|---|
| U | Phe | Ser | Tyr | Cys | U |
| | Phe | Ser | Tyr | Cys | C |
| | Leu | Ser | Non[2] | Non[3] | A |
| | Leu | Ser | Non[1] | Trp | G |
| C | Leu | Pro | His | Arg | U |
| | Leu | Pro | His | Arg | C |
| | Leu | Pro | Gln | Arg | A |
| | Leu | Pro | Gln | Arg | G |
| A | Ile | Thr | Asn | Ser | U |
| | Ile | Thr | Asn | Ser | C |
| | Ile | Thr | Lys | Arg | A |
| | Met | Thr | Lys | Arg | G |
| G | Val | Ala | Asp | Gly | U |
| | Val | Ala | Asp | Gly | C |
| | Val | Ala | Glu | Gly | A |
| | Val | Ala | Glu | Gly | G |

FIG. 29

FIG. 30

NUCLEOTIDE SEQUENCE OF A "LARGE TUMOR" GENE

```
CATGTCGAGGCTCAGCAGCTGAAAATTCAAACGCGCTAGTCAATGCTATCAATCTGTCGTGTTCACACGGCATCAAACGGTCACCAATGACGTCAATGGG
----.----+----.----+----.----+----.----+----.----+----.----+----.----+----.----+----.----+----.----+  100

TTTCCTTAGCTAATATAAAAACCAACGGCTCAGACTTACCAGCGGCAGGTATTTGTAGTACATCCAACACAGCGATGACGGTAGCTAATTGGCAGGTTCG
----.----+----.----+----.----+----.----+----.----+----.----+----.----+----.----+----.----+----.----+  200
                                                                            MetThrValAlaAsnTrpGlnValAr

AGATTTGACGCTTATCCTGCGCACCGGCGAGATGAAGAGTCGCTTGGAACAGGCGAGAACGGATTTCGGAGCGTTACTGTCCGAAACTGTATACTTTCAG
----.----+----.----+----.----+----.----+----.----+----.----+----.----+----.----+----.----+----.----+  300
gAspLevThrLevIleLevArgThrGlyGluMetLysSerArgLevGluGlnAlaArgThrAspPheGlyAlaLevLevSerGluThrValTyrPheGln

CCTTCCGCGATTAGACTTGGTGAGTTTGATGACGAGTACATCCACTCTCGACAAGAGCTGGCTTATGTATACCTTCGTGAAGATATTGCACGCCAATGCG
----.----+----.----+----.----+----.----+----.----+----.----+----.----+----.----+----.----+----.----+  400
ProSerAlaIleArgLevGlyGluPheAspAspGluTyrIleHisSerArgGlnGluLevAlaTyrValTyrLevArgGluAspIleAlaArgGlnCysA

CCTTGCGTCGAAACCTACCGTCCAACTCCTCTAACTTCGGAACAATGGCAACTGCAATACCGCCGTGGCTGATGAATGCACGCTGCCTGAATCGAGTTAT
----.----+----.----+----.----+----.----+----.----+----.----+----.----+----.----+----.----+----.----+  500
laLevArgArgAsnLevProSerAsnSerSerAsnPheGlyThrMetAlaThrAlaIleProProTrpLevMetAsnAlaArgCysLevAsnArgValMe

GCAGGAAAGGTGCGATCAAGGTGGCCTCGTCAACTACTATCAAGGCCCACATACAAATCAGTTCTTTTTGGCGATTATGCCAAGCAACTGCTTTGTTCGG
----.----+----.----+----.----+----.----+----.----+----.----+----.----+----.----+----.----+----.----+  600
tGlnGluArgCysAspGlnGlyGlyLevValAsnTyrTyrGlnGlyProHisThrAsnGlnPhePheLevAlaIleMetProSerAsnCysPheValArg

TTCGGGACCGACATAATCAACAATGAAAACTACGGTTTTAAGCCCGGGGAGGAATACACTAGAGGAAGGAGAAGATGACGACGATGAGATGGACGATGAA
----.----+----.----+----.----+----.----+----.----+----.----+----.----+----.----+----.----+----.----+  700
PheGlyThrAspIleIleAsnAsnGluAsnTyrGlyPheLysProGlyGluGluTyrThrArgGlyArgArgArgEnd

GGGGAGGCTGGTGGAGCGGAACCAAGAGAGTGTCAGATCGGAAACCTTATCAATTATCCGATCATTGCTTTAGGGTCATGCGATCTTTCCGCATAATTCC
----.----+----.----+----.----+----.----+----.----+----.----+----.----+----.----+----.----+----.----+  800

CGTCGCCGACACCTAATAAAGTCGGCTAATCTATGTGATTGAGTGTGTCTTGACTTTGTTATTTTGCATGTTTCCAATGTCATTTAGTAACGAAATAAAC
----.----+----.----+----.----+----.----+----.----+----.----+----.----+----.----+----.----+----.----+  900

GTTATCCTCTTCTAAAAGCAGGCTGTGTTTTCGGCAAACATCGCCACCCATCGCTAGTTTTTCTAAAAGTGTTCTAAGCTAGCATGGTAATAATCTATAC
----.----+----.----+----.----+----.----+----.----+----.----+----.----+----.----+----.----+----.----+  1000
```

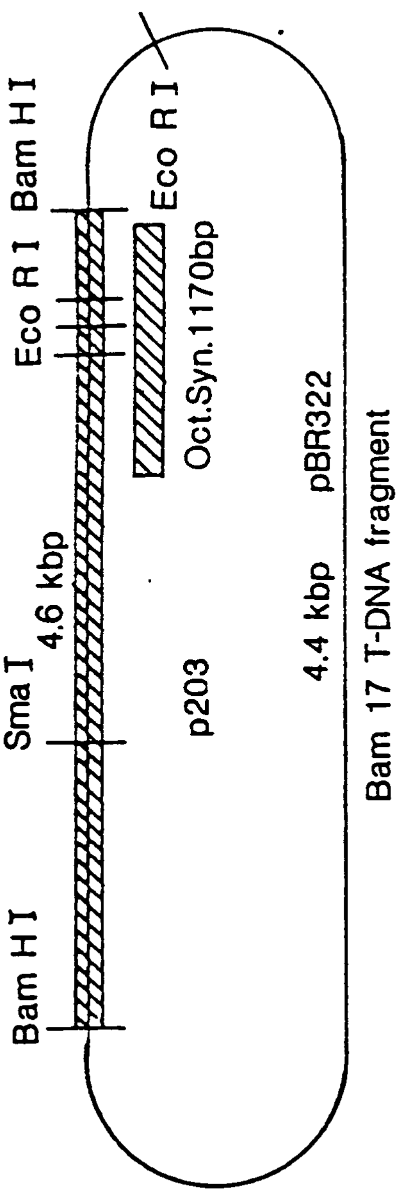

Bam H I
Eco R I
Bam H I
Eco R I
Sma I
4.6 kbp
Bam H I
Oct.Syn.1170bp
p203
pBR322
4.4 kbp
Bam 17 T-DNA fragment


FIG. 31

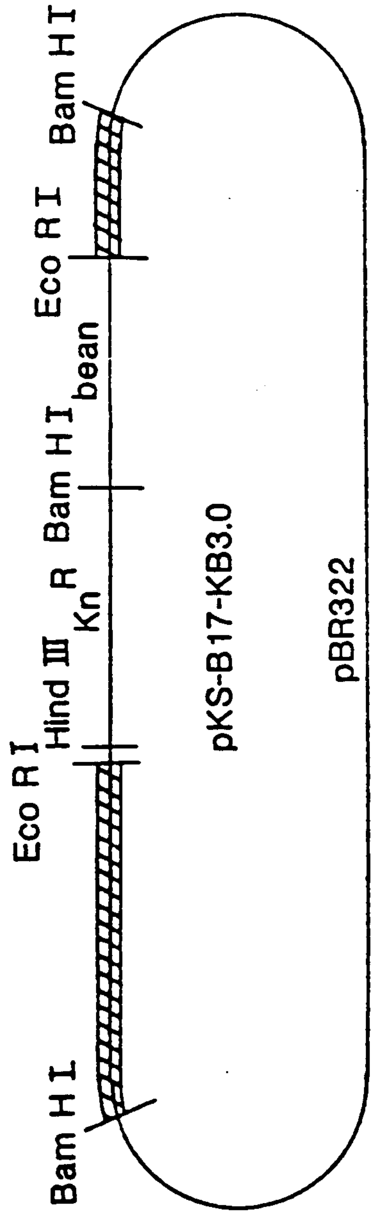

Bam H I
Eco R I
Bam H I bean
Kn R
Hind III
Eco R I
Bam H I
pKS-B17-KB3.0
pBR322


FIG. 32

FIG. 33-1

```
177.4   CATATGCGTGTCATCCCATGCCCAAATCTCCATGCATGTTCCAACCACCTTCTCTCTTATATAATACCTATAAATACCTCTAATATCACTCACTTCTTTC
        ---------+---------+---------+---------+---------+---------+---------+---------+---------+---------+  -1

177.4   ATCATCCATCCATCCAGAGTACTACTACTCTACTACTATAATACCCCAACCCAACTCATATTCAATACTACTCTACTATGATGAGAGCAAGGGTTCCACT
        ---------+---------+---------+---------+---------+---------+---------+---------+---------+---------+  100
cDNA31  ATCATCCATCCATCCAGAGTACTACTACTCTACTACTATAATACCCCAACCCAACTCATATTCAATACTACTCTACTATGATGAGAGCAAGGGTTCCACT
       /                 (5'-untranslated)                                            FMetMetArgAlaArgValProLe
  Cap
177.4   CCTGTTGCTGGGAATTCTTTTCCTGGCATCACTTTCTGCCTCATTTGCCACTTCACTCCGGGAGGAGGAAGAGAGCCAAGATAACCCCTTCTACTTCAAC
        ---------+---------+---------+---------+---------+---------+---------+---------+---------+---------+  200
cDNA31  CCTGTTGCTGGGAATTCTTTTCCTGGCATCACTTTCTGCCTCATTTGCCACTTCACTCCGGGAGGAGGAAGAGAGCCAAGATAACCCCTTCTACTTCAAC
        uLeuLeuLeuGlyIleLeuPheLeuAlaSerLeuSerAlaSerPheAlaThrSerLeuArgGluGluGluGluSerGlnAspAsnProPheTyrPheAsn

177.4   TCTGACAACTCCTGGAACACTCTATTCAAAAACCAATATGGTCACATTCGTGTCCTCCAGAGGTTCGACCAACAATCCAAACGACTTCAGAATCTTGAAG
        ---------+---------+---------+---------+---------+---------+---------+---------+---------+---------+  300
cDNA31  TCTGACAACTCCTGGAACACTCTATTCAAAAACCAATATGGTCACATTCGTGTCCTCCAGAGGTTCGACCAACAATCCAAACGACTTCAGAATCTTGAAG
        SerAspAsnSerTrpAsnThrLeuPheLysAsnGlnTyrGlyHisIleArgValLeuGlnArgPheAspGlnGlnSerLysArgLeuGlnAsnLeuGluA

177.4   ACTACCGTCTTGTGGAGTTCAGGTCCAAACCCGAAACCCTCCTTCTTCCTCAGCAGGCTGATGCTGAGTTACTCCTAGTTGTCCGTAGTGGTAAGTAATT
        ---------+---------+---------+---------+---------+---------+---------+---------+---------+---------+  400
cDNA31  ACTACCGTCTTGTGGAGTTCAGGTCCAAACCCGAAACCCTCCTTCTTCCTCAGCAGGCTGATGCTGAGTTACTCCTAGTTGTCCGTAGTG
        spTyrArgLeuValGluPheArgSerLysProGluThrLeuLeuLeuProGlnGlnAlaAspAlaGluLeuLeuLeuValValArgSerG

177.4   GCTACTGGTATCACTTGTTTCTTCTTGCAGAAATAATGGTAATGAGTTTTTTATAATTTCAGGGAGCGCCATACTCGTCTTGGTGAAACCTGATGATCGC
        ---------+---------+---------+---------+---------+---------+---------+---------+---------+---------+  500
cDNA31              (IVS 1, 72 bp)                                   GGAGCGCCATACTCGTCTTGGTGAAACCTGATGATCGC
                                                                     lySerAlaIleLeuValLeuValLysProAspAspArg

177.4   AGAGAGTACTTCTTCCTTACGAGCGATAACCCGATATTCTCTGATCACCAGAAAATCCCTGCAGGAACCATTTTCTATTTGGTTAACCCTGATCCCAAAG
        ---------+---------+---------+---------+---------+---------+---------+---------+---------+---------+  600
cDNA31  AGAGAGTACTTCTTCCTTACGAGCGATAACCCGATATTCTCTGATCACCAGAAAATCCCTGCAGGAACCATTTTCTATTTGGTTAACCCTGATCCCAAAG
        ArgGluTyrPhePheLeuThrSerAspAsnProIlePheSerAspHisGlnLysIleProAlaGlyThrIlePheTyrLeuValAsnProAspProLysG

177.4   AGGATCTCAGAATAATCCAACTCGCCATGCCCGTTAACAACCCTCAGATTCATGTACTGCCTTTTGTAATACCGAACTAATTTTTTGTTATTTTAACTTG
        ---------+---------+---------+---------+---------+---------+---------+---------+---------+---------+  700
cDNA31  AGGATCTCAGAATAATCCAACTCGCCATGCCCGTTAACAACCCTCAGATTCAT                                        (IVS 2,
        luAspLeuArgIleIleGlnLeuAlaMetProValAsnAsnProGlnIleHis

177.4   CAATTTCTCTCCAAATGTGATGATAAATGTTTGTCCTGTAGGAATTTTTCCTATCTAGCACAGAAGCCCAACAATCCTACTTGCAAGAGTTCAGCAAGCA
        ---------+---------+---------+---------+---------+---------+---------+---------+---------+---------+  800
cDNA31  88 bp)                                          GAATTTTTCCTATCTAGCACAGAAGCCCAACAATCCTACTTGCAAGAGTTCAGCAAGCA
                                                        GluPhePheLeuSerSerThrGluAlaGlnGlnSerTyrLeuGlnGluPheSerLysHi

177.4   TATTCTAGAGGCCTCCTTCAATGTAAGAAAGAAAACAGCATCTAACTACATATTTGCGTTGCCATTTAGCTAGTACTTTGTCTAAATGTCACACTTGTTG
        ---------+---------+---------+---------+---------+---------+---------+---------+---------+---------+  900
cDNA31  TATTCTAGAGGCCTCCTTCAAT                                                  (IVS 3, 124 bp)
        sIleLeuGluAlaSerPheAsn

177.4   AATTTGTTGAATGATATCATTATATATGTTTGCATGATTTTTATAGAGCAAATTCGAGGAGATCAACAGGGTTCTGTTTGAAGAGGAGGGACAGCAAGAG
        ---------+---------+---------+---------+---------+---------+---------+---------+---------+---------+  1000
cDNA31                                                       AGCAAATTCGAGGAGATCAACAGGGTTCTGTTTGAAGAGGAGGGACAGCAAGAG
                                                             SerLysPheGluGluIleAsnArgValLeuPheGluGluGluGlyGlnGlnGlu

177.4   GGAGTGATTGTGAACATTGATTCTGAACAGATTAAGGAACTGAGCAAACATGCAAAATCTAGTTCAAGGAAATCCCTTTCCAAACAAGATAACACAATTG
        ---------+---------+---------+---------+---------+---------+---------+---------+---------+---------+  1100
cDNA31  GGAGTGATTGTGAACATTGATTCTGAACAGATTAAGGAACTGAGCAAACATGCAAAATCTAGTTCAAGGAAATCCCTTTCCAAACAAGATAACACAATTG
        GlyValIleValAsnIleAspSerGluGlnIleLysGluLeuSerLysHisAlaLysSerSerSerArgLysSerLeuSerLysGlnAspAsnThrIleG

177.4   GAAACGAATTTGGAAACCTGACTGAGAGGACCGATAACTCCTTGAATGTGTTAATCAGTTCTATAGAGATGGAAGAGGTAAATACAAAGAAAAACCATAT
        ---------+---------+---------+---------+---------+---------+---------+---------+---------+---------+  1200
cDNA31  GAAACGAATTTGGAAACCTGACTGAGAGGACCGATAACTCCTTGAATGTGTTAATCAGTTCTATAGAGATGGAAGAG
        lyAsnGluPheGlyAsnLeuThrGluArgThrAspAsnSerLeuAsnValLeuIleSerSerIleGluMetGluGlu

177.4   AGACAAACTCAGCAATTGAGTTCTATTATTCACTGTCGTCTTGGTTAGAAAATCTTAGTATTGAGACTATAATTAAATAATGGTTTTTTTTGTTAACAAA
        ---------+---------+---------+---------+---------+---------+---------+---------+---------+---------+  1300
                          (IVS 4, 128 bp)

177.4   TTTAGGGAGCTCTTTTTGTGCCACACTACTATTCTAAGGCCATTGTTATACTAGTGGTTAATGAAGGAGAAGCACATGTTGAACTTGTTGGCCCAAAAGG
        ---------+---------+---------+---------+---------+---------+---------+---------+---------+---------+  1400
cDNA31       GGAGCTCTTTTTGTGCCACACTACTATTCTAAGGCCATTGTTATACTAGTGGTTAATGAAGGAGAAGCACATGTTGAACTTGTTGGCCCAAAAGG
             GlyAlaLeuPheValPheHisTyrTyrSerLysAlaIleValIleLeuValValAsnGluGlyGluAlaHisValGluLeuValGlyProLysGl

177.4   AAATAAGGAAACCTTGGAATATGAGAGCTACAGAGCTGAGCTTTCTAAAGACGATGTATTTGTAATCCCAGCAGCATATCCAGTTGCCATCAAGGCTACC
        ---------+---------+---------+---------+---------+---------+---------+---------+---------+---------+  1500
cDNA31  AAATAAGGAAACCTTGGAATATGAGAGCTACAGAGCTGAGCTTTCTAAAGACGATGTATTTGTAATCCCAGCAGCATATCCAGTTGCCATCAAGGCTACC
        yAsnLysGluThrLeuGluTyrGluSerTyrArgAlaGluLeuSerLysAspAspValPheValIleProAlaAlaTyrProValAlaIleLysAlaThr

177.4   TCCAACGTGAATTTCACTGGTTTCGGTATCAATGCTAATAACAACAATAGGAACCTCCTTGCAGGTATATATATTTATTATATATGACCATGAATTTGAA
        ---------+---------+---------+---------+---------+---------+---------+---------+---------+---------+  1600
cDNA31  TCCAACGTGAATTTCACTGGTTTCGGTATCAATGCTAATAACAACAATAGGAACCTCCTTGCAG
        SerAsnValAsnPheThrGlyPheGlyIleAsnAlaAsnAsnAsnAsnArgAsnLeuLeuAlaG

177.4   TATAGGGTTGTTGATGGAATTTTTTATTTATAATTGGTAATGCGTGATTGTGATTGTAAATATGAAGGTAAGACGGACAATGTCATAAGCAGCATCGGTA
        ---------+---------+---------+---------+---------+---------+---------+---------+---------+---------+  1700
cDNA31      (IVS 5, 103 bp)                                              GTAAGACGGACAATGTCATAAGCAGCATCGGTA
                                                                         lyLysThrAspAsnValIleSerSerIleGlyA
```

# FIG. 33-2

```
177.4   GAGCTCTGGACGGTAAAGACGTGTTGGGGCTTACGTTCTCTGGGTCTGGTGACGAAGTTATGAAGCTGATCAACAAACAGAGTGGATCGTACTTTGTGGA
        ---------+---------+---------+---------+---------+---------+---------+---------+---------+---------+  1800
cDNA31  GAGCTCTGGACGGTAAAGACGTGTTGGGGCTTACGTTCTCTGGGTCTGGTGACGAAGTTATGAAGCTGATCAACAAACAGAGTGGATCGTACTTTGTGGA
        rgAlaLeuAspGlyLysAspValLeuGlyLeuThrPheSerGlySerGlyAspGluValMetLysLeuIleAsnLysGlnSerGlySerTyrPheValAs

177.4   TGCACACCATCACCAACAGGAACAGCAAAAGGGAAGAAAGGGTGCATTTGTGTACTGAATAAGTATGAACTAAAATGCATGTAGGTGTAAGAGCTCATGG
        ---------+---------+---------+---------+---------+---------+---------+---------+---------+---------+  1900
cDNA31  TGCACACCATCACCAACAGGAACAGCAAAAGGGAAGAAAGGGTGCATTTGTGTACTGAATAAGTATGAACTAAAATGCATGTAGGTGTAAGAGCTCATGG
        pAlaHisHisHisGlnGlnGluGlnGlnLysGlyArgLysGlyAlaPheValTyrTER

177.4   AGAGCATGGAATATTGTATCCGACCATGTAACAGTATAATAACTGAGCTCCATCTCACTTCTTCTATGAATAAACAAAGGATGTTATGAT
        ---------+---------+---------+---------+---------+---------+---------+---------+---------+---------+  2000
cDNA31  AGAGCATGGAATATTGTATCCGACCATGTAACAGTATAATAACTGAGCTCCATCTCACTTCTTCTATGAATAAACAAAGGATGTTATGAT---Poly(A)
```

The complete sequence of a phaseolin gene and a cDNA